# EUROPEAN PATENT SPECIFICATION

(11) **EP 3 984 991 B1**
(45) Date of publication and mention of the grant of the patent: **18.06.2025**
(21) Application number: 20886173.2
(22) Date of filing: 18.11.2020
(51) Int. Cl.: C07C 231/12, C07C 235/80, B01J 31/22, C07D 273/04, C07C 239/18

(54) **METHOD FOR SYNTHESIZING ATORVASTATIN CALCIUM INTERMEDIATE BY USING MULTI-COMPONENT ONE-POT METHOD**
VERFAHREN ZUR MEHRKOMPONENTEN-EINTOPFSYNTHESE VON ZWISCHENPRODUKTEN VON ATORVASTATIN-CALCIUM
PROCÉDÉ DE SYNTHÈSE D'UN INTERMÉDIAIRE CALCIQUE D'ATORVASTATINE À L'AIDE D'UN PROCÉDÉ MONOTOPE À COMPOSANTS MULTIPLES

(30) Priority: 07.09.2020 CN 202010926970
(43) Date of publication of application: 20.04.2022
(73) Proprietor: Zhejiang Hongyuan Pharma Co., Ltd, Taizhou, Zhejiang 317016 (CN)
(72) Inventor: WANG, Haibo, Taizhou, Zhejiang 317016 (CN); MEI, Guangyao, Taizhou, Zhejiang 317016 (CN); FANG, Zhenrong, Taizhou, Zhejiang 317016 (CN); JIN, Hui, Taizhou, Zhejiang 317016 (CN); KUANG, Hongfu, Taizhou, Zhejiang 317016 (CN); ZHANG, Wei, Taizhou, Zhejiang 317016 (CN); QIU, Jinfu, Taizhou, Zhejiang 317016 (CN); XU, Chenli, Taizhou, Zhejiang 317016 (CN); LIU, Zhanyu, Taizhou, Zhejiang 317016 (CN); LIU, Xuesheng, Taizhou, Zhejiang 317016 (CN)
(74) Representative: Long, Giorgio
(86) International application number: PCT/CN2020/129673
(87) International publication number: WO 2022/048025

(56) References cited:
- WO-A1-02/057274
- WO-A1-2012/143933
- WO-A1-2012/143933
- US-A1- 2008 280 970
- RAO LI , LIU FENG-SI , GUAN SHI-LONG , ZHOU KAI : "Research on the synthesis of 4- (4-Fluorophenyl) -2 (2-Methylpropanoyl) -4-Oxo-N,beta-Diphenylbutanamide", JOURNAL OF WUHAN INSTITUTE OF TECHNOLOGY, vol. 32, no. 12, 15 December 2010 (2010-12-15), pages 10 - 12, XP055837877, ISSN: 1674-2869, DOI: 10.3969/j.issn.1674-2869.2010.12.003

## Description

The application claims the priority to Chinese Patent Application No. 202010926970.X, titled "METHOD FOR MULTICOMPONENT ONE-POT SYNTHESIS OF INTERMEDIATES OF ATORVASTATIN CALCIUM", filed on September 7, 2020 with the China National Intellectual Property Administration.

### FIELD

The present disclosure belongs to the technical field of organic chemistry and medicinal chemistry, and relates to a method for multicomponent one-pot synthesis of an intermediate of atorvastatin calcium. More specifically, the present disclosure relates to a one-pot synthesis of 4-(4-fluorophenyl)-2-(2-methylpropionyl)-3-phenyl-4-oxo-N-phenylbutanamide.

### BACKGROUND

Atorvastatin calcium, chemical name: bis{(3R,5R)-7-[2-(4-fluorophenyl)-5- (1-methylethyl)-3-phenyl-4-phenylaminocarbonyl-1H-pyrrol-1-yl]-3,5-dihydroxyheptanoic acid} calcium salt trihydrate, is an oral hydroxymethylglutaryl coenzyme A (HMG-CoA) reductase inhibitor, developed and successfully commercialized by Pfizer. This drug was first approved in the United States in February 1997 for the treatment of adult hypercholesterolemia. It reduces cholesterol and lipoprotein levels in plasma by inhibiting the synthesis of HMG-CoA reductase and cholesterol in the liver, and enhances uptake and metabolism of low-density lipoprotein (LDL) by increasing liver LDL receptors on the cell surface. Therefore, it can effectively inhibit the synthesis of endogenous cholesterol, and is effective for primary hypercholesterolemia, hypertriglyceridemia, familial hypercholesterolemia and mixed hyperlipidemia. At present, this drug for uses in many clinical indications in cardiovascular aspects has been approved for marketing in more than 20 countries and regions around the world, and has gained global annual sales up to more than 13 billion U.S. dollars. 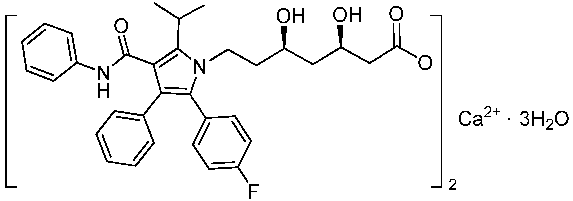 Atorvastatin calcium trihydrate

Owing to the huge market prospects of the generic drugs of atorvastatin calcium, scientific research institutions and pharmaceutical companies around the world have carried out a lot of researches on its preparation processes. At present, more than 100 patents on the process of atorvastatin calcium preparation are available. Although there have been a large number of reports on the synthetical technology of atorvastatin calcium, the current mainstream synthesis method is still the typical Paal-Knorr pyrrole synthesis, and it is also the mainstream production process most suitable for industrial production. 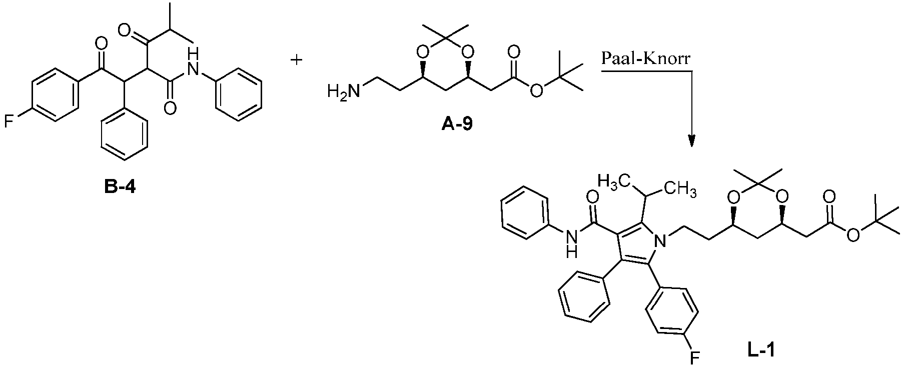 Paal-Knorr pyrrole synthesis of atorvastatin tert-butyl ester

The Paal-Knorr pyrrole synthesis of atorvastatin calcium mainly refers to two key intermediates, namely 4-(4-fluorophenyl)-2-(2-methylpropionyl)-3-phenyl-4-oxo-N-phenyl butanamide (hereinafter referred to as "B-4") and t-butyl [(4R,6R)-6-(2-aminoethyl)-2,2- dimethyl-1,3-dioxan-4-yl]acetate (hereinafter referred to as"A-9"). By Paal-Knorr pyrrole synthesis method, these two key intermediates construct the main structure of atorvastatin calcium, namely atorvastatin tert-butyl ester (hereinafter referred to as "L-1"), and then by deprotection of acetonylidene, alkali hydrolysis, salt-forming and other reactions, atorvastatin calcium is obtained. For the key intermediate 4-(4-fluorophenyl)-2-(2-methylpropionyl) -3-phenyl-4-oxo-N-phenylbutanamide (B-4), there have been numbers of reports on its synthesis.

In 1992, a method for synthesizing the intermediate 4-(4-fluorophenyl)-2- (2-methylpropionyl)-3-phenyl-4-oxo-N-phenylbutanamide (B-4) has been firstly reported by Butler et al. In this method, B-4 is obtained by 1,4-addition of 4-fluorobenzaldehyde with α,β-unsaturated ketone under the action of a thiazolcarbenes catalyst through Stetter reaction. But this type of catalyst is not only difficult to obtain, but also costly, severely malodorous, and environmentally unfriendly.

Subsequently, many studies on Stetter reaction catalysts have been carried out and various catalysts were developed. For example, in patent US2004072893, Sumitra et al. have disclosed using isobutyryl chloride, Mildren's acid and aniline as starting materials to synthesize isobutyrylacetanilide through a multicomponent reaction; then performing an Aldol condensation reaction to prepare α,β-unsaturated ketone, and finally reacting it with 4-fluorobenzaldehyde through a Stetter reaction to synthesize B-4.

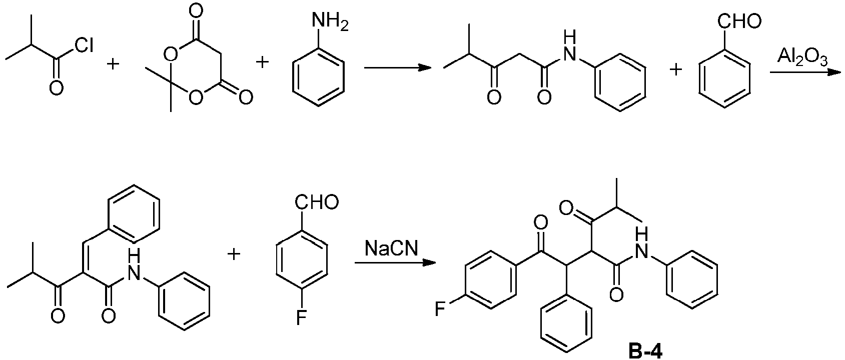

The raw materials of the process are cheap and easy to obtain, but the yield of the multicomponent reaction in the first step is not ideal; secondly, the highly toxic reagent NaCN is used in the process, which has a high EHS risk and large wastewater pollution.

In the patent WO2012143933, Jagapathi et al. have disclosed another method for synthesizing the key intermediate B-4. This process used phenylacetic acid as a raw material and undergos acid chloride preparation, Friedel-Crafts reaction, bromination reaction and substitution reaction in totally 4-steps to systhesize B-4 as shown below.

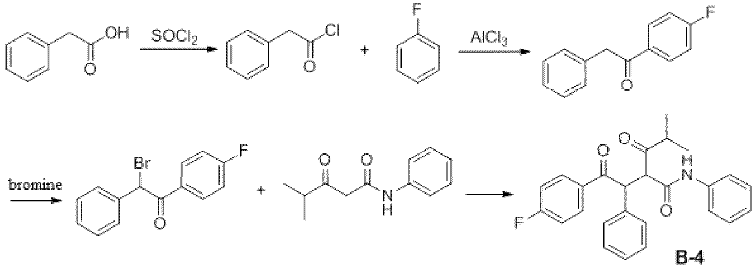

Although this process scheme uses cheap and easily available raw materials, and has high yield overall, it still has many defects: (1) the preparation of phenylacetyl chloride releases a greater odor, and uses a high-activity SOCl₂ as a chlorinating reagent, and a large amount of HCl and SO₂ gases and many acidic waste gases generate in this process, which is not environmentally friendly; (2) bromine atoms are introduced first and removed then during the process, which is poor in atom economy; (3) the Friedel-Crafts reaction also produces a large amount of acid gas HCl and large heat is generated during the quenching process, an EHS risk is high, and much wastewater containing salts are generated; and (4) although the process has avoided the use of expensive metal catalysts, the use of bromo reagent liquid bromine is strongly irritating and corrosive, and environmentally unfriendly. The international application WO 2012/143933 A1 discloses a process for preparing 4-fluoro-α-[2-methyl-1-oxopropyl]-γ-oxo-n-β-diphenylbenzene butane amide via a route which involves Friedel-Crafts, bromination and substitution reaction, but it does not describe the synethesis route of the intermediate of atorvastatin calcium of this application.

In view of the various defects of the existing technology, the inventors have developed a green and atomically economical method for multicomponent one-pot synthesis of 4-(4-fluorophenyl)-2-(2-methylpropionyl)-3-phenyl-4-oxo-N-phenylbutanamide. This method uses a catalytic amount of a transition metal-ligand catalyst, has high selectivity, and has obvious advantages over existing multi-step synthesis in yield. Moreover, the catalyst ligands can be simply synthesized, using conventional and classic organic reactions, and are relatively easy to obtain at a fully controllable cost. This multicomponent one-pot process is fully in line with the characteristics of the green chemical process, has advantages such as atomic economy, low emissions of the three-waste and is a preoparation process for synthesizing 4-(4-fluorophenyl)-2-(2-methylpropionyl)-3-phenyl-4-oxo-N-phenylbutanamide suitable for industrialization.

### SUMMARY

The present disclosure provides a method for multicomponent one-pot synthesis of an intermediate of atorvastatin calcium, and more specifically provides a method for multicomponent one-pot synthesis of 4-(4-fluorophenyl)-2-(2-methylpropionyl)-3-phenyl -4-oxo-N-phenylbutanamide. This process has simple steps, produces high yield, and is simple in operation, and is suitable for industrial production.

The method for multicomponent one-pot synthesis of 4-(4-fluorophenyl)-2-(2-methylpropionyl)-3-phenyl-4-oxo-N-phenylbutanamide described in the present disclosure includes the following solutions.

Provided is a method for multicomponent one-pot synthesis of an intermediate of atorvastatin calcium comprising:
reacting a compound of Formula II with 4-fluorobenzaldehyde and isobutyraldehyde under the action of Cu(SbF₆)₂ and a catalyst A by a one-pot process to synthesize a compound of Formula I: 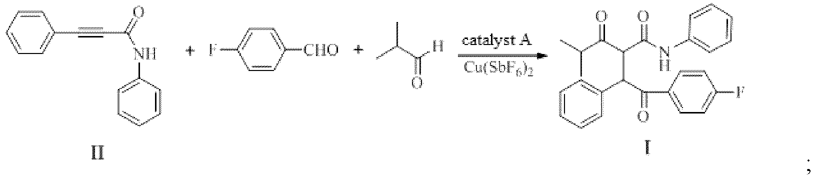 wherein the multicomponent reaction is carried out in an aprotic polar solvent which is one selected from tetrahydrofuran and N,N-dimethylformamide, or a mixed solvent thereof.

The method of the present disclosure is characterized in that: in the multicomponent reaction, the catalyst A is a combination of an N-hydroxyaminocyclohexylformamide derivative ligand and PdCl₂, and the N-hydroxyaminocyclohexylformamide derivative ligand has a structure represented by Formula III: 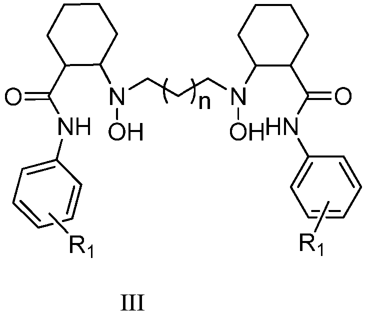
wherein, n is an integer from 1-4 natural numbers;
R₁ is an electron donating group selected from methyl, methoxy, and dimethylamino.

The method of the present disclosure is characterized in that: in the multicomponent reaction, the N-hydroxyaminocyclohexylformamide derivative ligand in the catalyst A is synthetized by the following scheme:
(1) reacting N-tert-butoxycarbonylaminocyclohexyl-2-carboxylic acid with a substituted aromatic amine under the action of a condensing agent to prepare compound IV;
(2) removing a Boc protection from the compound IV under an acidic condition to obtain compound V;
(3) reacting the compound V with a dibromoalkane-based substance to obtain compound VI; and
(4) reacting the compound VI under the action of an oxidizing agent to obtain an N-hydroxyaminocyclohexylformamide derivative ligand; 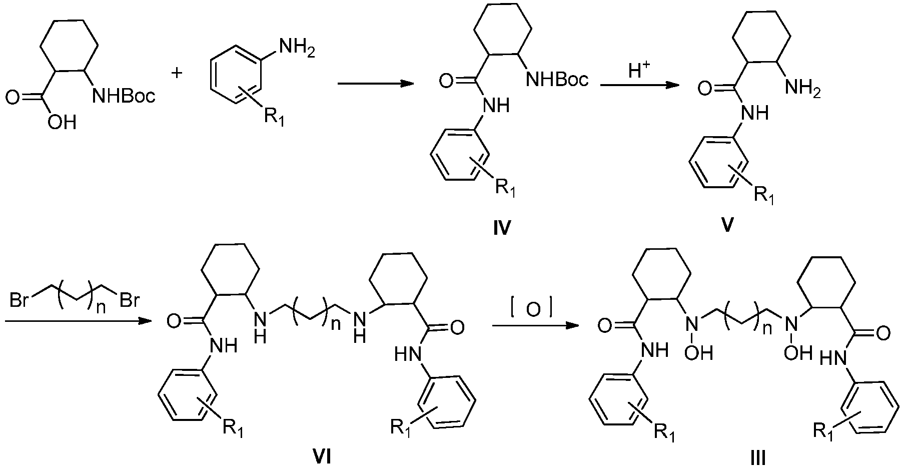
   wherein, n is an integer from 1-4 natural numbers;
   R₁ is an electron donating group selected from methyl, methoxy, and dimethylamino.

The method described in the present disclosure is characterized in that: in the multicomponent reaction, in the synthesis of the N-hydroxyaminocyclohexylformamide derivative ligand in the catalyst A, the condensing agent is selected from 1-propylphosphonic acid cyclic anhydride, 1-ethyl-(3-dimethylaminopropyl)carbodiimide hydrochloride, and dicyclohexylcarbodiimide; and the oxidizing agent is selected from hydrogen peroxide, tert-butyl hydroperoxide, and meta-chloroperoxybenzoic acid.

Furthermore, the method of the present disclosure is characterized in that: in the multicomponent reaction, a molar ratio of the N-hydroxyaminocyclohexylformamide derivative ligand to PdCl₂ in the catalyst A is 1.2-1.3 : 1.0.

Yet furthermore, the method of the present disclosure is characterized in that: in the multicomponent reaction, an amount of the catalyst A is from 0.005 to 0.008 molar equivalent relative to the compound of Formula II; an amount of Cu(SbF₆)₂ is from 0.001 to 0.003 molar equivalent relative to the compound of Formula II; an amount of 4-fluorobenzaldehyde is from 1.1 to 1.3 molar equivalent relative to the compound of Formula II; and an amount of isobutyraldehyde is from 1.5 to 2.5 molar equivalent relative to the compound of Formula II.

The method of the present disclosure is characterized in that: the multicomponent reaction is carried out under heating at a temperature from 50 to 60°C.

It should to be noted that the catalyst A of the present disclosure is a coordination compound formed by a ligand represented by Formula III and a transition metal. The present disclosure only describes the application of PdCl₂ and the ligands represented by Formula III, which are more effective and less expensive, in the multicomponent reaction of the present disclosure.

It should be further noted that for the ligand in the catalyst A of the present disclosure, that is, the compound represented by Formula III, R₁ in its structure is an electron donating group selected from methyl, methoxy, and dimethylamino. The present invention only claims to the electron donating group, mainly based on the consideration of the best effect. When R₁ is an electron withdrawing group, the catalyst A also has a certain catalytic effect on the multicomponent reaction in the present disclosure.

It should be yet further noted that for the ligand in the catalyst A of the present disclosure, that is, the compound represented by Formula III, R₁ in its structure is a meta electron donating substituent, and when n = 2, its catalytic effect and its selectivity are significantly improved. The possible reason is that the coordination compound formed by the transition metal Pd and the ligand forms a pocket-shaped cavity, which may effectively wrap the three reactants in the cavity for selective catalytic reaction, as shown in the following scheme:

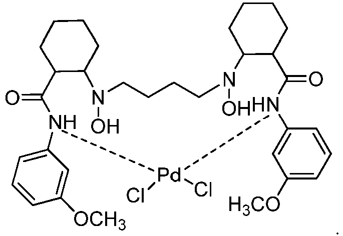

It should be emphasized and explained that the multicomponent reaction of the present disclosure is a synergistic reaction. If only the compound of Formula II is reacted with 4-fluorobenzaldehyde under the action of Cu(SbF₆)₂ and catalyst A, and then isobutyraldehyde is added for the reaction, 4-(4-fluorophenyl)-2-(2-methylpropionyl)-3-phenyl-4-oxo- N-phenylbutanamide will not be obtained. In this case, only the addition product of the compound of Formula II and 4-fluorobenzaldehyde will be observed. Similarly, if only the compound of Formula II is reacted with isobutyraldehyde under the action of Cu(SbF₆)₂ and catalyst A, and then 4-fluorobenzaldehyde is added, the target product will also not be obtained. Instead, only the addition product of the compound of Formula II and isobutyraldehyde will be observed. Besides, in these two cases, the regioselectivity of the addition of the aldehyde group to the carbon-carbon triple bond will significantly deteriorate.

The multicomponent reaction of the present disclosure is a synergistic reaction, which is also reflected in its high chemical selectivity. The reaction of the compound of Formula II with 4-fluorobenzaldehyde and isobutyraldehyde under the action of Cu(SbF₆)₂ and catalyst A has extremely high regioselectivity, and the formation of the compound of formula VII has not been observed. For the result of regioselectivity, those skilled in the present invention believe that probably it may be the steric hindrance that leads the way of a synergistic reaction only. The steric hindrance of the aniline group makes it difficult for the larger 4-fluorobenzaldehyde to approach the carbon end of the carbon-carbon triple bond thereof, while the smaller isobutyraldehyde is easier to participate in the reaction from the direction of the carbon-carbon triple bond of the aniline group, so that the reaction has a very high regioselectivity.

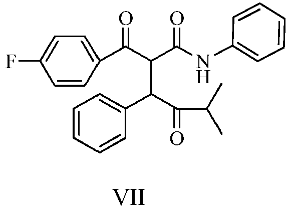

It should be further emphasized and explained that in the multicomponent reaction of the present disclosure, both Cu(SbF₆)₂ and the catalyst A are indispensable.

It should be yet further emphasized and explained that the multicomponent reaction catalyzed by Cu(SbF₆)₂ and catalyst A of the present disclosure needs to be carried out under anhydrous conditions. The presence of a certain amount of water will cause the reaction to fail to proceed smoothly, the presence of water will cause the hydrolysis of Cu(SbF₆)₂, and this would lead to catalyst deactivation and then the failure to proceed the reaction smoothly. The water content in the multicomponent reaction system catalyzed by Cu(SbF₆)₂ and the catalyst A of the present disclosure is preferably controlled not to be more than 200 ppm. When the moisture content is high, the reaction may be promoted by supplementing the catalyst, but a large use of the catalyst will cause the process to lose its cost advantage. The examples of the present disclosure are only the optimal embodiments based on cost considerations, but do not limit the present disclosure.

The amount of 4-fluorobenzaldehyde and the amount of isobutyraldehyde of the present disclosure are both preferred embodiments, but do not limit the present disclosure. Any adjustment and optimization of the feeding ratio will be obvious to those skilled in the art, and all should be considered to be included in the present disclosure. But, it needs to be emphasized that more excess isobutyraldehyde is necessary for the multicomponent reaction described in the present disclosure. Otherwise, the yield of the target product will be significantly reduced, and the addition product of the compound of Formula II and 4-fluorobenzaldehyde will be significantly increased.

The multicomponent reaction described in the present disclosure is carried out in an aprotic polar solvent which is one selected from tetrahydrofuran and N,N-dimethylformamide, or a mixed solvent thereof. The solvent is very important for the smooth progress of the multicomponent reaction described in the present disclosure. In other conventional aprotic solvents, the production of the target product has not been observed. The multicomponent reaction described in the present disclosure is carried out under the condition of heating to 50-60°C, which is a preferred embodiment provided by the present disclosure. This is because a higher reaction temperature may cause more side reactions, and a lower temperature may cause a large number of incompletely reacted compounds of Formula II.

Compared with the existing techniques, the multicomponent one-pot synthesis of 4-(4-fluorophenyl)-2-(2-methylpropionyl)-3-phenyl-4-oxo-N-phenylbutanamide described in the present disclosure has the following advantages and characteristics.
(1) The multicomponent one-pot synthesis of 4-(4-fluorophenyl)-2-(2-methylpropionyl) -3-phenyl-4-oxo-N-phenylbutanamide described in the present disclosure is in line with the characteristics of green chemistry and high atom economy, and has significantly reduced the emission of the three-waste and pollution factors.
(2) The multicomponent one-pot synthesis of 4-(4-fluorophenyl)-2-(2-methylpropionyl) -3-phenyl-4-oxo-N-phenylbutanamide described in the present disclosure comprises fewer reaction steps, and has a significantly higher yield (approximately 80%-87%) than that of the existing multi-step synthesis technical solutions (its total yield is approximately 63%-66%).
(3) The multicomponent one-pot synthesis of 4-(4-fluorophenyl)-2-(2-methylpropionyl) -3-phenyl-4-oxo-N-phenylbutanamide described in the present disclosure uses readily available raw materials, all of which are simple chemical raw materials or may be synthesized with high yield using simple chemical raw materials, and has simple process operations, a low risk in EHS, and high industrial practicability.

### Terms:

The term "multicomponent reaction" described in the present disclosure, abbreviated as MCR, refers to a process of forming a new compound containing the main structural fragments of all components from three or more compounds through a one-pot reaction. The multicomponent reaction process involves at least two functional groups, which may be regarded as a combination of multiple bimolecular reactions. It is not just a sum of several bimolecular reactions in quantity, but is an orderly reaction that must be carried out according to Domilo rule. Since such reaction is mainly based on addition, the atoms lost during the reaction are less than that of the substitution reaction, which is more in line with the principle of atom economy. Compared with traditional bimolecular reactions, multicomponent reactions have great advantages in generating molecular structure complexity and diversity.

The term "ligand" described in the present disclosure refers to atoms, molecules and ions that can bond with the central atom (metals or metalloids). Generally speaking, a ligand will provide at least one electron when participating in the bonding. The ligand acts as Lewis base. In the ligand, the atom that can provide a lone pair electrons to directly form a coordination bond with the central atom is called a coordinating atom, such as C in CO. A coordinating atom contains lone pair electrons in the outermost electron shell. The most common ones are atoms of non-metallic elements with greater electronegativity, such as N, O, C, S, and halogens.

The term "transition metals" described in the present disclosure refers to a series of metallic elements in the d-block and ds-block of the periodic table (The elements in the d-block include elements of groups IIIB-VIIB and VIII of the periodic table, but do not include the lanthanides and actinides. The ds-block includes elements of groups IB-IIB of the periodic table). Owing to the underfilled d orbitals in the valence shell, transition metals have distinctly different properties from other elements based on the 18-electron rule. Due to the presence of empty d orbitals, transition metals can easily accept foreign electrons and form coordination compounds with ligands that can provide a lone pair of electrons.

The term "coordination compound" described in the present disclosure refers to a class of compounds with a characteristic chemical structure, formed by fully or partly bonding a central atom (or ion, collectively referred to as central atom) and surrounding molecules or ions (called as ligands) through coordinate bonds. A coordination compound is generally a compound formed by bonding a transition metal atom or ion with the molecules (e.g., CO, NH₃, H₂O) or ions (e.g., Cl⁻, CN⁻, NO²⁻) containing a lone pair of electrons through coordinate bonds. In a coordination compound, the central atom and the ligand share two electrons. The formed chemical bond is called a coordination bond. The two electrons are not provided by each of the two atoms, but by the ligand atom itself.

### DETAILED DESCRIPTION

The following specific examples enable those skilled in the art to fully understand the present disclosure.

In the following examples, unless otherwise specified, all temperatures were the degrees Celsius. Unless otherwise specified, the room temperature was 20-30°C. Unless otherwise specified, all starting materials and reagents were commercially available, and were used directly without further purification. Unless otherwise specified, all solvents were industrial-grade solvents and were used directly without further treatment. Unless otherwise specified, commercially available manufacturers include but are not limited to Hangzhou Chemical Reagent, Sinopharm Reagent, etc.

In the following examples, unless otherwise specified, all the HPLC analysis were performed under the following conditions and using the following methods: using a Venusil XBP-C18 chromatographic column with 4.6 × 150 mm, 5 µm or a similar chromatographic column; at flow rate of 1.0 mL/min; with an injection volume of 10 µL; at a column temperature of 25°C; at a wavelength of 246 nm; for a run time of 40 min; using acetonitrile and purified water at a ratio of 3 : 2 as a mobile phase; using acetonitrile (one drop of phosphoric acid was added to 100 mL of acetonitrile) as a diluent; the samples were prepared by weighing out about 25 mg of a sample in a 25 mL of volumetric flask and diluting it with the diluent to make a 25 mL solution. The peak time of 4-(4-fluorophenyl)-2-(2-methylpropionyl)-3-phenyl-4-oxo-N- phenylbutanamide was about 12 min.

### Example 1: Preparation of a ligand of Formula III

### Preparation of compound IV-1:

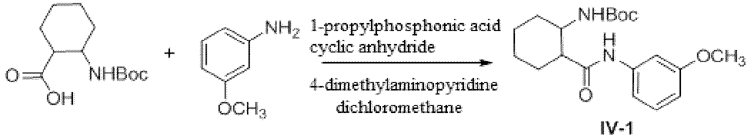

10 g of N-tert-butoxycarbonylaminocyclohexyl-2-carboxylic acid (41 mmol), 5.5 g of m-methoxyaniline (44.7 mmol, 1.08 eq) and 50 mL of dichloromethane were added into a 250 ml reaction flask, and the mixture was stirred and cooled to 0-10°C, and then 15.7 g of 1-propylphosphonic acid cyclic anhydride (49 mmol, 1.2 eq), 1.5 g of 4-dimethylaminopyridine (12 mmol, 0.3 eq), and 9.1 g of triethylamine (90 mmol, 2.2 eq) were added successively. After the addition was finished, the mixture was kept at a temperature of 0-10°C and stirred to react for 8 h. After the reaction was completed, the reaction liquid was concentrated until dry under reduced pressure. 45 mL of isopropanol was added to the concentrate, and the mixture was stirred until it was dissolved and clear, and then 15 mL of water was added dropwise to crystallize with stirring at 0-10°C for 2 h. The mixture was filtered. The filter cake was dried under reduced pressure at 35-45°C for 6-10 h, to obtain 13.6 g of compound IV-1 at a yield of 95.1% with an HPLC purity of 98.6%. ESI-MS: m/z 349.31 [M+H]⁺; ¹H-NMR(125MHz, CD₃Cl): δ 9.30(br s, 1H), 8.66 (br s, 1H), 7.78(s, 1H), 7.38(dd, 1H), 7.26 (d, 1H), 6.97(d, 1H), 3.56 (s, 3H), 3.44-3.48(m, 1H), 3.07-3.11 (m, 1H), 1.65-1.88(m, 4H), 1.51-1.54 (m, 2H), 1.37(s, 9H), 1.13-1.17(m, 2H).

### Preparation of compound V-1:

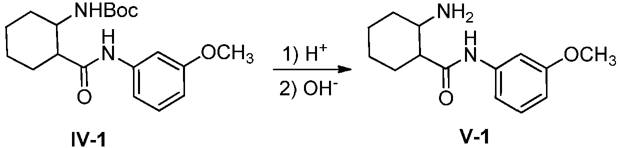

10 g of the compound IV-1 (28 mmol) and 50 mL of tetrahydrofuran were added into a 250 mL reaction flask and the mixture was stirred until it was dissolved and clear. 63 mL of 5 N HCl solution (31 mmol, 1.1 eq) was added dropwise to the dissolved and clear solution. After the addition was finished, the mixture was stirred to react at a temperature of 25-35°C for 6 h. After the reaction was completed, NaOH was added dropwise to adjust the pH to 9-10. The mixture was extracted with 50 mL of toluene for 3 times. The toluene phases were combined together and concentrated under reduced pressure until dry. 20 mL of dichloromethane was added to the concentrate, and the mixtutre was stirred until it was dissolved and clear. 200 ml of methyl tert-butyl ether was added dropwise to the dissolved and clear solution. The resulting suspension was stirred for 2 h and filtered. The filter cake was dried under reduced pressure at 35-45°C for 4-6 h, to obtain 6.8 g of a dry target product V-1 at a yield of 95.5% with a HPLC purity of 98.0%. ESI-MS: m/z 249.03 [M+H]⁺; ¹H-NMR(125MHz, CD₃Cl): δ 8.62(br s, 1H), 7.76 (s, 1H), 7.37(dd, 1H), 7.25 (d, 1H), 6.98(d, 1H), 5.02(br s, 2H), 3.56 (s, 3H), 3.45-3.48(m, 1H), 3.08-3.12(m, 1H), 1.66-1.88(m, 4H), 1.50-1.54 (m, 2H), 1.12-1.16 (m, 2H).

### Preparation of compound VI-1:

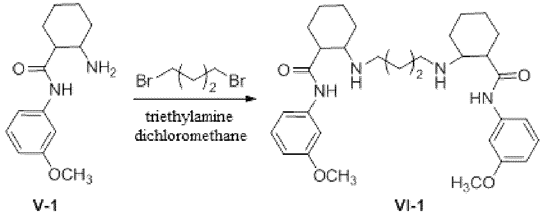

6.5 g of the compound V-1 (0.026 mol), 33 mL of dichloromethane and 5.51 g of triethylamine (0.055 mol, 2.1 eq) were added into a 100 mL reaction flask, and the mixture was stirred, and cooled to 0-10°C. To the cooled mixture, 2.35 g of 1,4-dibromobutane (0.011 mol, 0.42 eq) was added dropwise slowly. After the addition was finished, the reaction liquid was heated up to 20-30°C and kept at that temperature to react for 2 h. After the reaction was completed, the reaction liquid was cooled to 5-15°C, and 20 mL of water was added dropwise thereto to quench the reaction. The reaction liquid was allowed to stand for phase separation and an organic phase was separated out. The organic phase was concentrated under reduced pressure at 35-45°C. After the concentration was completed, a slurry-like liquid was obtained. To the slurry-like liquid, 20 mL of dichloromethane was added, and the mixture was then stirred until it was dissolved and clear. 200 mL of methyl tert-butyl ether was then added dropwise to the dissolved and clear solution. The resulting suspension was stirred for 2 h at 20-30°C and filtered. The filter cake was dried at 40-50°C under reduced pressure for 6 h, to obtain 5.54 g of the compound VI-1 at a yield of 92.3% with a HPLC purity of 99.3%. ESI-MS: m/z 551.61[M+H]⁺; H-NMR(125MHz, CD₃Cl): δ 8.60(br s, 2H), 7.74 (s, 2H), 7.35 (dd, 2H), 7.22(d, 2H), 6.88(d, 2H), 5.06 (br s, 2H), 3.55 (s, 6H), 3.42-3.48(m, 2H), 3.06-3.12(m, 2H), 2.60-2.68(m, 4H), 1.62-1.88(m, 8H), 1.40-1.56 (m, 8H), 1.10-1.16 (m, 4H).

### Preparation of a compound III-1:

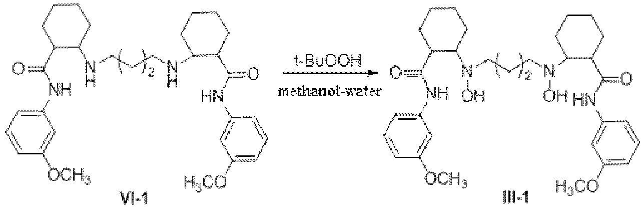

5.3 g of the compound VI-1 (0.010 mol) was weighed out and added into a 100 mL reaction flask, and 30 mL of methanol and 30 mL of water were then added, and the mixture was stirred until it was dissolved and clear. After the dissolved and clear solution was cooled to 10-15°C, 1.48 g of 70% tert-butyl hydroperoxide (0.011 mol, 1.1 eq) was added dropwise to the cooled dissolved and clear solution. After the addition was finished, the solution was heated up to 30-35°C to react for 15 h, to obtain a light yellow suspension. The suspension was filtered. The filter cake was washed with 30 mL of water, and dried at 50-60°C under reduced pressure for 10 h, to obtain a total of 4.99 g of the compound III-1 at a yield of 89.8% with a purity of 99.5%. ESI-MS: m/z 583.39[M+H]⁺; H-NMR(125MHz, CD₃Cl): δ 8.56 (br s, 2H), 7.75 (s, 2H), 7.36 (dd, 2H), 7.20(d, 2H), 6.86 (d, 2H), 4.58(br s, 2H), 3.56 (s, 6H), 3.40-3.48(m, 2H), 3.05-3.12(m, 2H), 2.61-2.68(m, 4H), 1.62-1.86 (m, 8H), 1.40-1.56 (m, 8H), 1.12-1.16 (m, 4H).

### Example 2: Preparation of a ligand of Formula III

### Preparation of compound IV-2:

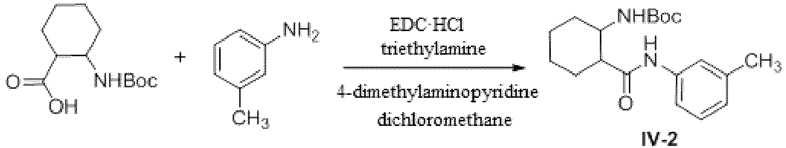

10 g of N-tert-butoxycarbonylaminocyclohexyl-2-carboxylic acid (41 mmol), 4.75 g of meta-toluidine (44.3 mmol, 1.08 eq) and 50 mL of dichloromethane were added into a 250 mL reaction flask, and the mixture was stirred and cooled to 0-10°C. To the reaction mixture, 17.3 g of 1-ethyl-(3-dimethylaminopropyl)carbodiimide hydrochloride (90 mmol, 2.2 eq), 1.5 g of 4-dimethylaminopyridine (12 mmol, 0.3 eq), and 9.1 g of triethylamine (90 mmol, 2.2 eq) were added. After the addition was finished, the mixture was kept at a temperature of 0-10°C and stirred to react for 8 h. After the reaction was completed, the reaction liquid was concentrated until dry under reduced pressure. To the concentrate, 45 mL of isopropanol was added, and the mixture was stirred until it was dissolved and clear. To the dissolved and clear solution, 15 mL of water was added dropwise, and the mixture was stirred at 0-10°C for 2 h, and filtered. The filter cake was dried at 35-45°C under reduced pressure for 6-10 h, to obtain 13.0 g of the compound IV-2 at a yield of 95.5% with a HPLC purity of 98.1%. ESI-MS: m/z 333.20 [M+H]⁺; ¹H-NMR(125MHz, CD₃Cl): δ 8.91 (br s, 1H), 8.56 (br s, 1H), 7.71 (s, 1H), 7.31 (dd, 1H), 7.22(d, 1H), 6.72(d, 1H), 3.42-3.47(m, 1H), 3.06-3.10(m, 1H), 2.21 (s, 3H), 1.66-1.86 (m, 4H), 1.50-1.55 (m, 2H), 1.38(s, 9H), 1.12-1.18(m, 2H).

### Preparation of compound V-2:

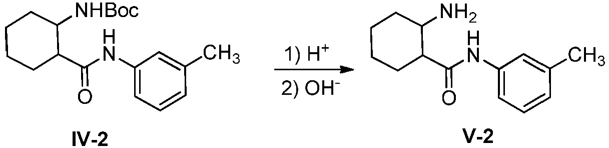

10 g of the compound IV-2 (30 mmol) and 50 mL of tetrahydrofuran were added to a 250 mL reaction flask and and the mixture was stirred until it was dissolved and clear. To the dissolved and clear solution, 66 mL of a 0.5 N HCl solution (33 mmol, 1.1 eq) was added dropwise. After the addition was finished, the mixture was stirred to react at 25-35°C for 6 h. After the reaction was completed, NaOH was added dropwise to adjust the pH to 9-10. The mixture was extracted with 50 mL of toluene for 3 times. The toluene phases were combined together, and concentrated at 50-60°C under reduced pressure until dry. To the concentrate, 20 mL of dichloromethane was added, and the mixture was stirred until it was dissolved and clear. To the dissolved and clear solution, 200 mL of methyl tert-butyl ether was added dropwise, and the mixture was stirred for 2 h, and filtered. The filter cake was dried under reduced pressure at 35-45°C for 4-6 h, to obtain 6.6 g of the target product V-2 at a yield of 95.0% with a HPLC purity of 98.5%. ESI-MS: m/z 232.16 [M+H]⁺; ¹H-NMR(125MHz, CD₃Cl): δ 8.60(br s, 1H), 7.72(s, 1H), 7.30(dd, 1H), 7.21 (d, 1H), 6.76 (d, 1H), 5.21 (br s, 2H), 3.42-3.48(m, 1H), 3.05-3.10(m, 1H), 2.20 (s, 3H), 1.65-1.86 (m, 4H), 1.51-1.55 (m, 2H), 1.12-1.17(m, 2H).

### Preparation of compound VI-2:

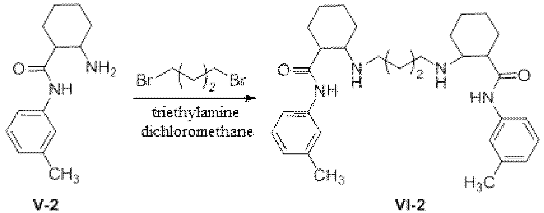

6.3 g of the compound V-2 (0.027 mol), 32 mL of dichloromethane and 5.71 g of triethylamine (0.056 mol, 2.1 eq) were added into a 100 mL reaction flask, and the mixture was stirred and cooled to 0-10°C. To the cooled mixture, 2.44 g of 1,4-dibromobutane (0.011 mol, 0.41 eq) was added dropwise. After the addition was finished, the reaction liquid was heated up to 20-30°C to react for 2 h. After the reaction was completed, the reaction liquid was cooled to 5-15°C. To the cooled reaction liquid, 20 mL of water was added dropwise to quench the reaction. The reaction liquid was allowed to stand for phase separation and an organic phase was separated out. The organic phase was separated out, and concentrated at 35-45°C under reduced pressure to obtain a slurry-like liquid. To the slurry-like liquid, 20 mL of dichloromethane was added, and the mixture was stirred until it was dissolved and clear. 200 mL of methyl tert-butyl ether was then added dropwise to the dissolved and clear solution. The resulting suspension was stirred for 2 h at 20-30°C, and filtered. The filter cake was dried at 40-50°C under reduced pressure for 6 h, to obtain 5.48 g of the compound VI-2 at a yield of 93.5% with a HPLC purity of 99.5%. ESI-MS: m/z 519.44[M+H]⁺; ¹H-NMR(125MHz, CD₃Cl): δ 8.62(br s, 2H), 7.76 (s, 2H), 7.28(dd, 2H), 7.20(d, 2H), 6.78(d, 2H), 5.08(br s, 2H), 3.40-3.48(m, 2H), 3.02-3.10(m, 2H), 2.50-2.61 (m, 4H), 2.21 (s, 6H), 1.62-1.88(m, 8H), 1.46-1.56 (m, 8H), 1.12-1.18(m, 4H).

### Preparation of a compound III-2:

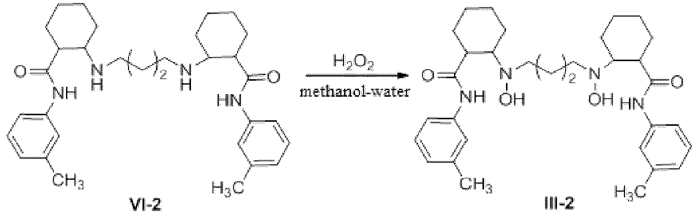

5.2 g of the compound VI-2 (0.010 mol) was weighed out and added into a 100 mL reaction flask, and 35 mL of methanol and 25 mL of water were then added. The mixture was stirred until it was dissolved and clear. 1.36 g of a 30% hydrogen peroxide solution (0.012 mol, 1.2 eq) was added dropwise to the dissolved and clear solution at 20-30°C. After the addition was finished, the mixture was heated up to 40-45°C to react for 16 h to obtain a light yellow suspension. The suspension was filtered. The filter cake was dried at 50-60°C under reduced pressure, to obtain 4.92 g of the compound III-2 at a yield of 89.3% with a purity of 99.6%. ESI-MS: m/z 551.73[M+H]⁺; ¹H-NMR(125MHz, CD₃Cl): δ 8.60(br s, 2H), 7.77(s, 2H), 7.26 (dd, 2H), 7.21 (d, 2H), 6.76 (d, 2H), 4.60(br s, 2H), 3.41-3.48(m, 2H), 3.03-3.10(m, 2H), 2.50-2.60(m, 4H), 2.21 (s, 6H), 1.60-1.88(m, 8H), 1.45-1.56 (m, 8H), 1.12-1.18(m, 4H).

### Example 3: Preparation of a ligand of Formula III

### Preparation of compound IV-3:

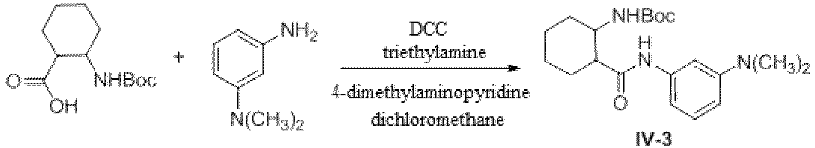

10 g of N-tert-butoxycarbonylaminocyclohexyl-2-carboxylic acid (41 mmol), 6.05 g of meta-dimethylaminoaniline (44.4 mmol, 1.08 eq) and 50 mL of dichloromethane were added to a 250 mL reaction flask, and the mixture was stirred until it was dissolved and clear. The dissolved and clear solution was cooled to 0-10°C, and 18.6 g of dicyclohexylcarbodiimide (90 mmol, 2.2 eq), 1.5 g of 4-dimethylaminopyridine (12 mmol, 0.3 eq), and 9.1 g of triethylamine (90 mmol, 2.2 eq) were added. After the addition was finished, the mixture were kept at a temperature of 0-10°C and stirred to react for 8 h. After the reaction was completed, the reaction liquid was concentrated until dry under reduced pressure. To the concentrate, 45 mL of isopropanol was added, and the mixture was stirred until it was dissolved and clear. To the dissolved and clear solution, 15 mL of water was added dropwise, and the mixture was stirred to crystallize at 0-10°C for 2 h, and filtered. The filter cake was dried at 35-45°C under reduced pressure for 6-10 h, to obtain 14.1 g of the compound IV-3 at a yield of 95.3% with a HPLC purity of 98.7%. ESI-MS: m/z 362.63 [M+H]⁺; ¹H-NMR(125MHz, CD₃Cl): δ 9.28(br s, 1H), 8.45 (br s, 1H), 7.77(s, 1H), 7.42(dd, 1H), 7.33(d, 1H), 6.98(d, 1H), 3.42-3.47(m, 1H), 3.10 (s, 6H), 3.08-3.13(m, 1H), 1.62-1.86 (m, 4H), 1.50-1.54 (m, 2H), 1.38(s, 9H), 1.13-1.18(m, 2H).

### Preparation of compound V-3:

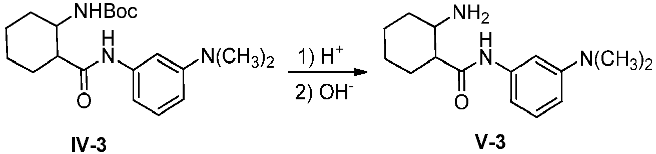

10 g of the compound IV-3 (27 mmol) and 50 mL of tetrahydrofuran were added to a 250 mL reaction flask, and the mixture was stirred until it was dissolved and clear. 60 mL of a 0.5 N HCl solution (30 mmol, 1.1 eq) was added dropwise to the dissolved and clear solution. After the addition was finished, the mixture was stirred at 25-35°C to react for 6 h. After the reaction was completed, NaOH was added dropwise to adjust the pH to 9-10. The mixture was extracted with 50 mL of toluene for 3 times. The toluene phases were combined together, and concentrated at 50-60°C under reduced pressure until dry. To the concentrate, 20 mL of dichloromethane was added, and the mixture was stirred until it was dissolved and clear. To the dissolved and clear solution, 200 mL of methyl tert-butyl ether was added dropwise. The resulting suspension was stirred for 2 h and filtered. The filter cake was dried under reduced pressure at 35-45°C for 4-6 h. 6.9 g, to obtain the target product V-3 at a yield of 95.2% with a HPLC purity of 98.3%. ESI-MS: m/z 262.43 [M+H]⁺; ¹H-NMR(125MHz, CD₃Cl): δ 8.50(br s, 1H), 7.76 (s, 1H), 7.40(dd, 1H), 7.31 (d, 1H), 6.96 (d, 1H), 5.16 (br s, 2H), 3.42-3.48(m, 1H), 3.11 (s, 6H), 3.06-3.10(m, 1H), 1.62-1.84 (m, 4H), 1.51-1.54 (m, 2H), 1.12-1.18(m, 2H).

### Preparation of compound VI-3:

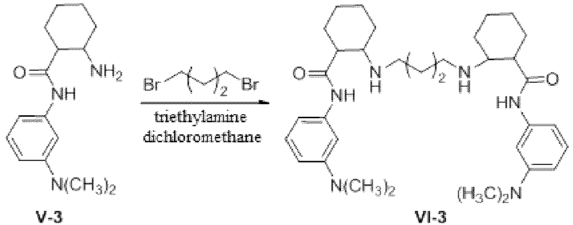

6.7 g of the compound V-3 (0.026 mol), 34 mL of dichloromethane and 5.39 g of triethylamine (0.053 mol, 2.0 eq) were added into a 100 mL reaction flask, and the mixture was stirred and cooled to 0-10°C. To the cooled reaction liquid, 2.31 g of 1,4-dibromobutane (0.011 mol, 0.42 eq) was added dropwise. After the addition was finished, the reaction mixture was heated up to 20-30°C to react for 2 h. After the reaction was completed, the reaction liquid was cooled to 5-15°C, and 20 mL of water was added dropwise to quench the reaction. The reaction liquid was allowed to stand for phase separation. The organic phase was separated out, and concentrated at 35-45°C under reduced pressure to obtain a slurry-like liquid. To the slurry-like liquid, 20 mL of dichloromethane was added, and the mixture was stirred until it was dissolved and clear. To the dissolved and clear solution, 200 mL of methyl tert-butyl ether was then added dropwise. After the addition was finished, the mixture was stirred at 20-30°C for 2 h and filtered. The filter cake was dried at 40-50°C under reduced pressure for 6 h. 5.66 g of the compound VI-3 was obtained at a yield of 91.9% with a HPLC purity of 99.0%. ESI-MS: m/z 577.63[M+H]⁺; ¹H-NMR(125MHz, CD₃Cl): δ 8.62(br s, 2H), 7.75 (s, 2H), 7.38(dd, 2H), 7.26 (d, 2H), 6.88(d, 2H), 5.08(br s, 2H), 3.40-3.48(m, 2H), 3.12 (s, 12H), 3.02-3.10(m, 2H), 2.48-2.56 (m, 4H), 1.60-1.86 (m, 8H), 1.42-1.56 (m, 8H), 1.12-1.18(m, 4H).

### Preparation of a compound III-3:

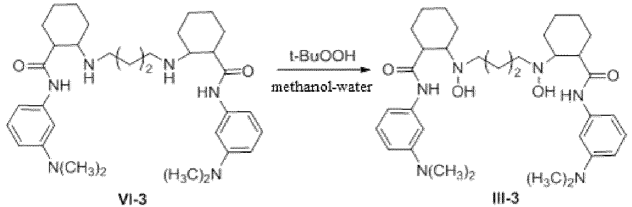

5.4 g of the compound VI-3 (0.009 mol) was weighed out and added into a 100 mL reaction flask. 30 mL of methanol and 30 mL of water were then added, and the mixture was stirred until it was dissolved and clear. The dissolved and clear solution was cooled to 10-15°C. To the cooled dissolved and clear solution, 1.44 g of 70% tert-butyl hydroperoxide (0.011 mol, 1.2 eq) was added dropwise. After the addition was finished, the mixture was heated up to 30-35°C to react for 15 h to obtain a light yellow suspension. The suspension was filtered. The filter cake was dried at 50-60°C under reduced pressure, to obtain 4.96 g of the compound III-3 at a yield of 87.6% with a purity of 99.5%. ESI-MS: m/z 609.63[M+H]⁺; ¹H-NMR(125MHz, CD₃Cl): δ 8.56 (br s, 2H), 7.76 (s, 2H), 7.35 (dd, 2H), 7.25 (d, 2H), 6.86 (d, 2H), 4.60(br s, 2H), 3.42-3.48(m, 2H), 3.11 (s, 12H), 3.02-3.10(m, 2H), 2.46-2.56 (m, 4H), 1.60-1.88(m, 8H), 1.40-1.56 (m, 8H), 1.11-1.18(m, 4H).

### Example 4: Preparation of a ligand of Formula III

### Preparation of compound IV-4:

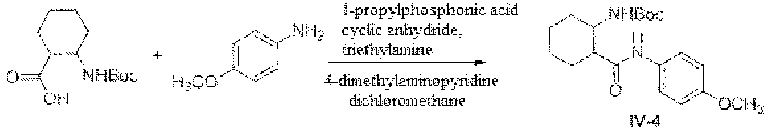

10 g of N-tert-butoxycarbonylaminocyclohexyl-2-carboxylic acid (41 mmol), 5.5 g of para-anisidine (44 mmol, 1.08 eq), and 50 mL of dichloromethane were added to a 250 mL reaction flask, and the mixture was stirred until it was dissolved and clear, and cooled to 0-10°C. To the dissolved and clear solution, 15.7 g of 1-propylphosphonic acid cyclic anhydride (49 mmol, 1.2 eq), 1.5 g of 4-dimethylaminopyridine (12 mmol, 0.3 eq) and 9.1 g of triethylamine (90 mol, 2.2 eq) were added. After the addition was finished, the mixture was kept at a temperature of 0-10°C and stirred to react for 8 h. After the reaction was completed, the reaction liquid was concentrated until dry under reduced pressure. To the concentrate, 45 mL of isopropanol was added, and the mixture was stirred until it was dissolved and clear. To the dissolved and clear solution, 15 mL of water was added dropwise. The mixture was stirred at 0-10°C to crystallize for 2 h, and filtered. The filter cake was dried at 35-45°C under reduced pressure for 8-10 h, to obtain 13.7 g of the compound IV-4 at a yield of 95.5% with a HPLC purity of 98.5%. ESI-MS: m/z 349.40 [M+H]⁺; ¹H-NMR(125MHz, CD₃Cl): 9.28(br s, 1H), 8.56 (br s, 1H), δ 7.78(d, 2H), 7.37(d, 2H), 3.55 (s, 3H), 3.45-3.49(m, 1H), 3.06-3.08(m, 1H), 1.64-1.88(m, 4H), 1.50-1.54 (m, 2H), 1.36 (s, 9H), 1.14-1.17(m, 2H).

### Preparation of compound V-4:

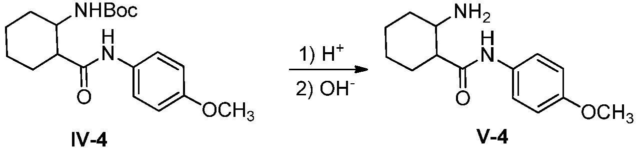

10 g of the compound IV-4 (28 mmol) and 50 mL of THF were added to a 250 mL reaction flask and the mixture was stirred until it was dissolved and clear. To the dissolved and clear solution, 63 mL of a 5 N HCl solution (31 mmol, 1.1 eq) was added dropwise. After the addition was finished, the reaction mixture was stirred at 25-35°C for 6 h. After the reaction was completed, NaOH was added dropwise to adjust the pH to 9-10. The mixture was extracted with 50 mL of toluene for 3 times. The toluene phases were combined together and concentrated at 50-60°C under reduced pressure until dry. To the concentrate, 20 mL of dichloromethane was added, and the mixture was stirred until it was dissolved and clear. To the dissolved and clear solution, 200 mL of methyl tert-butyl ether was added dropwise. The resulting suspension was stirred for 2 h and filtered. The filter cake was dried under reduced pressure at 35-45°C for 4-6 h, to obtain 6.8 g of the compound V-4 at a yield of 95.5% with a HPLC purity of 98.3%. ESI-MS: m/z 249.31 [M+H]⁺; ¹H-NMR(125MHz, CD₃Cl): δ 8.62(br s, 1H), 7.76 (d, 2H), 7.35 (d, 2H), 3.56 (s, 3H), 5.22(br s, 2H), 3.44-3.49(m, 1H), 3.06-3.11 (m, 1H), 1.64-1.86 (m, 4H), 1.51-1.54 (m, 2H), 1.14-1.18(m, 2H).

### Preparation of compound VI-4:

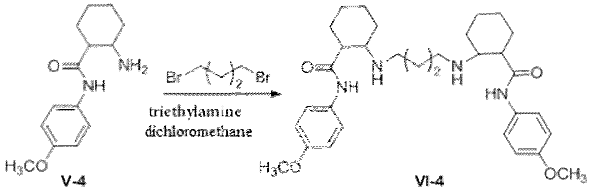

6.5 g of the compound V-4 (0.026 mol), 33 mL of dichloromethane and 5.51 g of triethylamine (0.055 mol, 2.1 eq) were added into a 100 mL reaction flask. The mixture was stirred, and cooled to 0-10°C. To the cooled reaction mixture, 2.35 g of 1,4-dibromobutane (0.011 mol, 0.42 eq) was added dropwise. After the addition was finished, the mixture was heated up to 20-30°C to react for 2 h. After the reaction was completed, the reaction liquid was cooled to 5-15°C, and 20 mL of water was added to quench the reaction. The reaction liquid was allowed to stand for phase separation. The organic phase was separated out, and concentrated at 35-45°C under reduced pressure to obtain a slurry-like liquid. To the slurry-like liquid, 20 mL of dichloromethane was added, and stirred until it was dissolved and clear. To the dissolved and clear solution, 200 mL of methyl tert-butyl ether was added dropwise. The resulting suspension was stirred at 20-30°C for 2 h and filtered. The filter cake was dried at 40-50°C under reduced pressure for 6 h, to obtain 5.56 g of the compound VI-4 at a yield of 92.6% with a HPLC purity of 98.5%. ESI-MS: m/z 551.46 [M+H]⁺; ¹H-NMR(125MHz, CD₃Cl): δ 8.56 (br s, 2H), 7.75 (d, 4H), 7.32(d, 4H), 3.57 (s, 6H), 5.20(br s, 2H), 3.40-3.49(m, 2H), 3.03-3.10(m, 2H), 2.52-2.60(m, 4H), 1.60-1.88(m, 8H), 1.44-1.56 (m, 8H), 1.12-1.18(m, 4H).

### Preparation of a compound III-4:

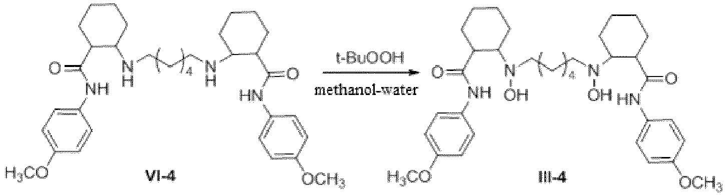

5.3 g of the compound VI-4 (0.010 mol) was weighed out and added into a 100 mL reaction flask, and 40 mL of methanol and 20 mL of water were added. The mixture was stirred until it was dissolved and clear. At a controled temperature of 10-20°C, 1.82 g of meta-chloroperoxybenzoic acid (0.011 mol, 1.1 eq) was added to the dissolved and clear solution in batches. After the addition was finished, the mixture was heated up to 25-35°C to react for 8 h to obtain a light yellow suspension. The suspension was filtered. The filter cake was dried at 50-60°C under reduced pressure. 4.82 g of the compound III-4 was obtained at a yield of 86.5% with a purity of 99.2%. ESI-MS: m/z 583.46[M+H]⁺; ¹H-NMR(125MHz, CD₃Cl): δ 8.60(br s, 2H), 7.76 (d, 4H), 7.33(d, 4H), 3.58 (s, 6H), 4.65 (br s, 2H), 3.40-3.48(m, 2H), 3.02-3.10(m, 2H), 2.51-2.60(m, 4H), 1.60-1.88(m, 8H), 1.42-1.56 (m, 8H), 1.12-1.18(m, 4H).

### Example 5: Preparation of a ligand of Formula III

### Preparation of compound IV-5:

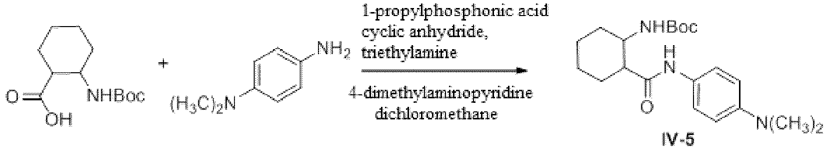

10 g of N-tert-butoxycarbonylaminocyclohexyl-2-carboxylic acid (41 mmol), 6.05 g of para-dimethylaminoaniline (44 mmol, 1.08 eq), and 50 mL of dichloromethane were added to a 250 ml reaction flask. The mixture was stirred until it was dissolved and clear, and cooled to 0-10°C. To the cooled dissolved and clear solution, 15.7 g of 1-propylphosphonic acid cyclic anhydride (49 mmol, 1.2 eq), 1.5 g of 4-dimethylaminopyridine (12 mmol, 0.3 eq), and 9.1 g of triethylamine (90 mol, 2.2 eq) were added. After the addition was finished, the mixture was kept at a temperature of 0-10°C and stirred to react for 8 h. After the reaction was completed, the reaction liquid was concentrated until dry under reduced pressure. To the concentrate, 45 mL of isopropanol was added, and the mixture was stirred until it was dissolved and clear. To the dissolved and clear solution, 15 mL of water was added dropwise. The mixture was stirred and crystallized at 0-10°C for 2 h, and filtered. The filter cake was dried at 35-45°C under reduced pressure for 8-12 h, to obtain 14.1 g of the compound IV-5 at a yield of 95.3% with a HPLC purity of 98.6%. ESI-MS: m/z 362.55 [M+H]⁺; ¹H-NMR(125MHz, CD₃Cl): 9.36 (br s, 1H), 8.50(br s, 1H), δ 7.77(d, 2H), 7.46 (d, 2H), 3.42-3.46 (m, 1H), 3.12 (s, 6H), 3.06-3.10(m, 1H), 1.62-1.80(m, 4H), 1.51-1.54 (m, 2H), 1.37(s, 9H), 1.14-1.18(m, 2H).

### Preparation of compound V-5:

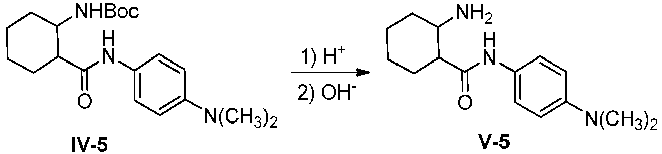

10 g of the compound IV-5 (27 mmol) and 50 mL of THF were added to a 250 ml reaction flask and the mixture was stirred until it was dissolved and clear. To the dissolved and clear solution, 60 mL of a 0.5 N HCl solution (30 mmol, 1.1 eq) was added dropwise. After the addition was finished, the reaction mixture was stirred at 25-35°C for 6 h. After the reaction was completed, NaOH was added dropwise to adjust the pH to 9-10. The mixture was extracted with 50 mL of toluene for 3 times. The toluene phases were combined together, and concentrated at 50-60°C under reduced pressure until dry. To the concentrate, 20 mL of dichloromethane was added, and the mixture was stirred until it was dissolved and clear. To the dissolved and clear solution, 200 mL of methyl tert-butyl ether was added dropwise. The resulting suspension was stirred for 2 h and filtered. The filter cake was dried under reduced pressure at 35-45°C for 4-6 h, to obtain 6.9 g of the target product V-5 at a yield of 95.2% with a HPLC purity of 98.6%. ESI-MS: m/z 262.61 [M+H]⁺; ¹H-NMR(125MHz, CD₃Cl): δ 8.66 (br s, 1H), 7.76 (d, 2H), 7.45 (d, 2H), 5.12(br s, 2H), 3.42-3.48(m, 1H), 3.11 (s, 6H), 3.05-3.10(m, 1H), 1.62-1.76 (m, 4H), 1.50-1.54 (m, 2H), 1.13-1.18(m, 2H).

### Preparation of compound VI-5:

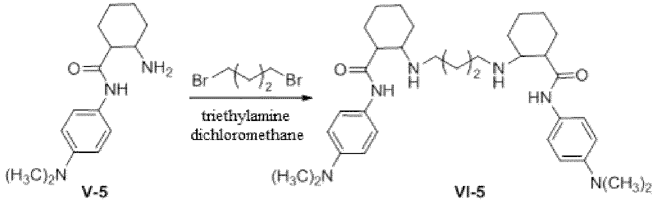

6.3 g of the compound V-5 (0.024 mol), 32 mL of dichloromethane and 5.07 g of triethylamine (0.050 mol, 2.1 eq) were added into a 100 mL reaction flask. The mixture was stirred, and cooled to 0-10°C. To the cooled reaction liquid, 2.17 g of 1,4-dibromobutane (0.010 mol) was added dropwise. After the addition was finished, the mixture was heated up to 20-30°C to react for 2 h. After the reaction was completed, the reaction liquid was cooled to 5-15°C, and 20 mL of water was added dropwise to quench the reaction. The reaction liquid was allowed to stand for phase separation. The organic phase was separated out and concentrated at 35-45°C to obtain a slurry-like liquid. To the slurry-like liquid, 15 mL of methyl chloride was added, and the mixture was stirred until it was dissolved and clear. 150 mL of methyl tert-butyl ether was added dropwise. The resulting suspension was stirred at 20-30°C for 2 h, and filtered. The filter cake was dried at 40-50°C under reduced pressure for 6 h, to obtain 5.32 g of the compound VI-5 at a yield of 91.8% with a HPLC purity of 98.4%. ESI-MS: m/z 577.73 [M+H]⁺; ¹H-NMR(125MHz, CD₃Cl): δ 8.62(br s, 2H), 7.73(d, 4H), 7.40(d, 4H), 5.06 (br s, 2H), 3.40-3.48(m, 2H), 3.12 (s, 12H), 3.01-3.08(m, 2H), 2.50-2.62(m, 4H), 1.60-1.78(m, 8H), 1.42-1.56 (m, 8H), 1.12-1.18(m, 4H).

### Preparation of a compound III-5:

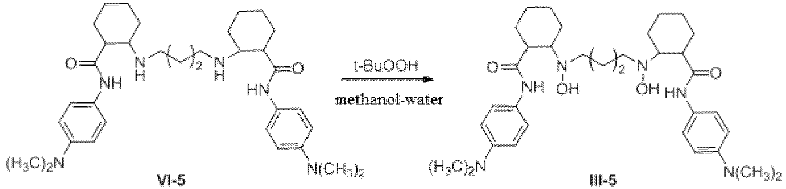

5.1 g of the compound VI-5 (0.009 mol) was weighed out and added into a 100 mL reaction flask, and 30 mL of methanol and 30 mL of water were added. The mixture was stirred until it was dissolved and clear. To the dissolved and clear solution, 1.18 g of 70% tert-butyl hydroperoxide (0.011 mol, 1.2 eq) was added dropwise at 10-15°C. After the addition was finished, the mixture was heated up to 30-35°C to react for 15 h to obtain a light yellow suspension. The suspension was filtered. The filter cake was dried at 50-60°C under reduced pressure, to obtain 4.70 g of the compound III-5 at a yield of 88.5% with a purity of 99.4%. ESI-MS: m/z 609.47[M+H]⁺; ¹H-NMR(125MHz, CD₃Cl): δ 8.60(br s, 2H), 7.70(d, 4H), 7.38(d, 4H), 4.55 (br s, 2H), 3.42-3.48(m, 2H), 3.10 (s, 12H), 3.01-3.07(m, 2H), 2.50-2.60(m, 4H), 1.61-1.78(m, 8H), 1.41-1.56 (m, 8H), 1.12-1.18(m, 4H).

### Example 6: Preparation of a ligand of Formula III

### Preparation of compound VI-6:

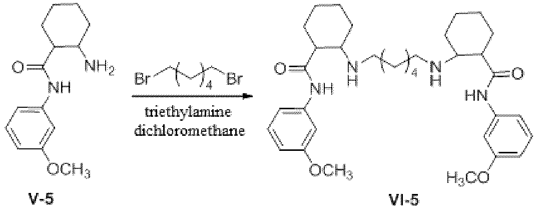

6.5 g of the compound V-1 (0.026 mol), 34 mL of dichloromethane and 6.07 g of triethylamine (0.060 mol, 2.3 eq) were added into a 100 mL reaction flask. The mixture was stirred, and cooled to 0-10°C. To the cooded reaction mixture, 2.93 g of 1,6-dibromohexane (0.012 mol, 0.46 eq) was added dropwise. After the addition was finished, the mixture was heated up to 20-30°C to react for 2 h. After the reaction was completed, the reaction liquid was cooled to 5-15°C, and 20 mL of water was added dropwise to quench the reaction. The reaction liquid was allowed to stand for phase separation. The organic phase was separated out and concentrated at 40-50°C under reduced pressure to obtain a slurry-like liquid. To the slurry-like liquid, 20 mL of dichloromethane was added, and the mixture was stirred until it was dissolved and clear. 200 mL of methyl tert-butyl ether was then added dropwise to the dissolved and clear solution. The resulting suspension was stirred at 20-30°C for 2 h and filtered. The filter cake was dried at 40-50°C under reduced pressure for 6 h, to obtain 6.30 g of the target product VI-6 at a yield of 90.7% with a HPLC purity of 96.6%. ESI-MS: m/z 579.70[M+H]⁺; H-NMR(125MHz, CD₃Cl): δ 8.62(br s, 2H), 7.74 (s, 2H), 7.36 (dd, 2H), 7.20(d, 2H), 6.89(d, 2H), 5.02(br s, 2H), 3.57 (s, 6H), 3.40-3.48(m, 2H), 3.06-3.13(m, 2H), 2.59-2.68(m, 4H), 1.62-1.86 (m, 8H), 1.40-1.60(m, 12H), 1.10-1.18(m, 4H).

### Preparation of a compound III-6:

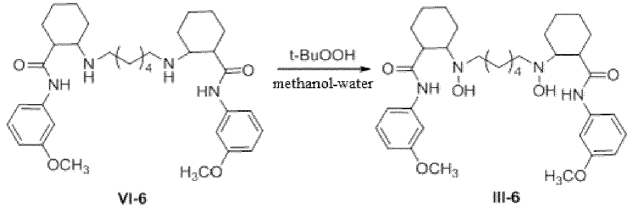

5.8 g of the compound VI-6 (0.010 mol) was weighed out and added into a 100 mL reaction flask, and 40 mL methanol and 20 mL of water were added. The mixture was stirred until it was dissolved and clear. At a controled temperature of 10-20°C, 1.97 g of meta-chloroperoxybenzoic acid (0.011 mol, 1.1 eq) was added in batches to the dissolved and clear solution. After the addition was finished, the mixture was heated up to 25-35°C to react for 8 h to obtain a light yellow suspension. The suspension was filtered. The filter cake was dried at 50-60°C under reduced pressure, to obtain 5.15 g of the compound III-6 at a yield of 84.5% with a purity of 98.4%. ESI-MS: m/z 611.38[M+H]⁺; ¹H-NMR(125MHz, CD₃Cl): δ 8.60(br s, 2H), 7.76 (s, 2H), 7.35 (dd, 2H), 7.21 (d, 2H), 6.86 (d, 2H), 4.50(br s, 2H), 3.56 (s, 6H), 3.41-3.48(m, 2H), 3.06-3.12(m, 2H), 2.60-2.68(m, 4H), 1.60-1.86 (m, 8H), 1.40-1.60(m, 12H), 1.10-1.18(m, 4H).

### Example 7: Preparation of a catalyst A

0.89 g of PdCl₂ (5.0 mmol) and 50 mL of anhydrous methanol were added into a 150 mL of round-bottomed flask, and the flask was replaced using nitrogen for 3 times. 3.50 g of the ligand III-1 (6.0 mmol, 1.2 eq) was then added to the round-bottomed flask, and the flask was replaced using nitrogen for 3 times. The resulting brown mixture was heated to reflux and stirred for 16 h until a large amount of insoluble solids precipitated. The suspension was cooled to room temperature, filtered, washed with 5 mL of anhydrous methanol, and dried under reduced pressure at 30-40°C to obtain 3.76 g of a yellow solid, namely catalyst A-1, at a yield of 95%.

### Example 8: Preparation of a catalyst A

0.89 g of PdCl₂ (5.0 mmol) and 50 mL of anhydrous methanol were added into a 150 mL of round-bottomed flask, and the flask was replaced using nitrogen for 3 times. 3.44 g of the ligand III-2 (6.3 mmol, 1.25 eq) was then added to the round-bottomed flask, and the flask was replaced using nitrogen for 3 times. The resulting brown mixture was heated to reflux and stirred for 16 h until a large amount of insoluble solids precipitated. The suspension was cooled to room temperature, filtered, washed with 5 mL of anhydrous methanol, and dried under reduced pressure at 30-40°C to obtain 3.54 g of a yellow solid, namely catalyst A-2, at a yield of 93%.

### Example 9: Preparation of a catalyst A

0.89 g of PdCl₂ (5.0 mmol) and 50 mL of anhydrous methanol were added into a 150 mL of round-bottomed flask, and the flask was replaced using nitrogen for 3 times. 3.64 g of the ligand III-3 (6.0 mmol, 1.2 eq) was then added to the round-bottomed flask, and the flask was replaced using nitrogen for 3 times. The resulting brown mixture was heated to reflux and stirred for 16 h until a large amount of insoluble solids precipitated. The suspension was cooled to room temperature, filtered, washed with 5 mL of anhydrous methanol, and dried under reduced pressure at 30-40°C to obtain 3.78 g of a yellow solid, namely catalyst A-3, at a yield of 93%.

### Example 10: Preparation of a catalyst A

0.89 g of PdCl₂ (5.0 mmol) and 50 mL of anhydrous methanol were added into a 150 mL of round-bottomed flask, and the flask was replaced using nitrogen for 3 times. 3.77 g of the ligand III-4 (6.5 mmol, 1.3 eq) was then added to the round-bottomed flask, and the flask was replaced using nitrogen for 3 times. The resulting brown mixture was heated to reflux and stirred for 16 h until a large amount of insoluble solids precipitated. The suspension was cooled to room temperature, filtered, washed with 5 mL of anhydrous methanol, and dried under reduced pressure at 30-40°C to obtain 3.51 g of a yellow solid, namely catalyst A-4, at a yield of 89%.

### Example 11: Preparation of a catalyst A

0.89 g of PdCl₂ (5.0 mmol) and 50 mL of anhydrous methanol were added into a 150 mL of round-bottomed flask, and the flask was replaced using nitrogen for 3 times. 3.94 g of the ligand III-5 (6.5 mmol, 1.3 eq) was then added to the round-bottomed flask, and the flask was replaced using nitrogen for 3 times. The resulting brown mixture was heated to reflux and stirred for 16 h until a large amount of insoluble solids precipitated. The suspension was cooled to room temperature, filtered, washed with 5 mL of anhydrous methanol, and dried under reduced pressure at 30-40°C to obtain 3.54 g of a yellow solid, namely catalyst A-5, at a yield of 87%.

### Example 12: Preparation of a catalyst A

0.89 g of PdCl₂ (5.0 mmol) and 50 mL of anhydrous methanol were added into a 150 mL of round-bottomed flask, and the flask was replaced using nitrogen for 3 times. 3.95 g of the ligand III-6 (6.5 mmol, 1.3 eq) was then added to the round-bottomed flask, and the flask was replaced using nitrogen for 3 times. The resulting brown mixture was heated to reflux and stirred for 16 h until a large amount of insoluble solids precipitated. The suspension was cooled to room temperature, filtered, washed with 5 mL of anhydrous methanol, and dried under reduced pressure at 30-40°C to obtain 3.67 g of a yellow solid, namely catalyst A-6, at a yield of 90%.

### Example 13: Preparation of the compound of Formula II

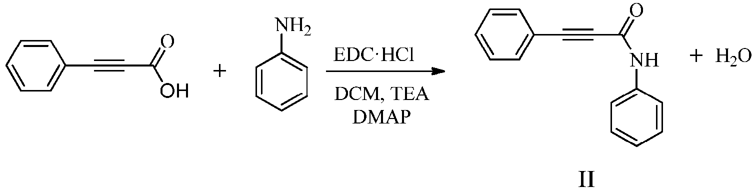

To a 5 L three-necked flask, 500 g of phenylpropiolic acid (3.42 mol), 334 g of aniline (3.59 mol, 1.05 eq), and 2.5 L of dichloromethane were added successively, and the mixture was stirred until it was dissolved and clear. The dissolved and clear solution was cooled to 0-10°C. 691 g of triethylamine (6.84 mol, 2.0 eq), 1.45 Kg of 1-ethyl-(3-dimethylaminopropyl) carbodiimide hydrochloride (7.53 mol, 2.2 eq) and 125 g of 2-dimethylaminopyridine (1.03 mol, 0.3 eq) were added successively. After the addition was finished, the reaction liquid was heated up to 20-30°C and stirred for 16 hours.

After the reaction was detected by TLC, the reaction liquid was washed by sequentially adding 1 L of a 0.05% hydrochloric acid solution, 1 L of water, and 1 L of a saturated sodium chloride solution thereto. The washed dichloromethanethe organic phase was concentrated under reduced pressure until dry at 40-50°C.

To the concentrate, 750 mL of dichloromethane was added, and the mixture was stirred until it was dissolved and clear. The dissolved and clear solution was then transferred into a 20 L glass reaction kettle. 10 L of methyl tert-butyl ether was added dropwise to the dissolved and clear solution to precipitate out a white solid. The resulting suspension was stirred at 20-30°C for 1 hour, and filtered. The product was washed with 1 L of methyl tert-butyl ether, and dried, to obtain 708 g of a white power, namely the compound of Formula II, with a HPLC purity of 99.3% at a molar yield of 93.5%. ESI-MS: m/z 223.37 [M+H]⁺; ¹H-NMR(125MHz, CD₃Cl): δ 9.55 (br s, 1H), 7.71 (d, 2H), 7.56 (d, 2H), 7.47 (dd, 2H), 7.45 (dd, 2H), 7.41 (d, 1H), 7.17 (d, 1H).

### Example 14: Preparation of 4-(4-fluorophenyl)-2-(2-methylpropionyl)-3-phenyl -4-oxoN-phenylbutanamide

To a 250 mL three-necked flask, 10.0 g of the compound of Formula II (0.045 mol), 6.45 g of 4-fluorobenzaldehyde (0.052 mol, 1.15 eq) and 5.86 g of isobutyraldehyde (0.08 mol, 1.8 eq) were added successively, and then 80 mL of tetrahydrofuran was added. The mixture was stirred until it was dissolved and clear. The gas in the flask was replaced with nitrogen for 3 times. 36 mg of Cu(SbF₆)₂ (0.068 mmol, 0.0015 eq) and 196 mg of a catalyst A-1 (0.25 mmol, 0.0055 eq) were added successively to the dissolved and clear solution, and the mixture was stirred until it was dissolved and clear again. The gas in the flask was replaced with nitrogen for 3 times. The flask was installed with a condenser tube, at the top of which a nitrogen bulb was equipped. The mixture was heated up to 50-60°C to react for 24 h.

The heating was stopped, and the reaction liquid was cooled to room temperature. 10 mL of a saturated ammonium chloride solution was added dropwise to quench the reaction. The quenched reaction liquid was concentrated at 45-55°C under reduced pressure to remove away tetrahydrofuran. To the concentrated residue, 80 mL of dichloromethane was added, and the mixture was stirred until it was dissolved and clear. The mixture was subjected to phase separation to remove the saturated ammonium chloride aqueous solution. The dichloromethane layer was then washed with 50 mL of water and the water layer was discarded. The dichloromethane layer was further washed with 50 mL of saturated brine and the brine layer was discarded.

0.5 g of silica gel was added to the dichloromethane layer, and the mixture was stirred for 30 minutes to remove residual heavy metals, and filtered. The filter residue was washed with 30 mL of dichloromethane for twice, and the dichloromethane filtrate was combined. The dichloromethane filtrate was added with 50 mL of isopropanol, and concentrated at 45-50°C under normal pressure to remove dichloromethane. The resulting suspension was cooled to 0-10°C, stirred for 1 hour, and filered. The wet cake was dired, to obtain 16.5 g of a white powder with a HPLC purity of 99.8% and a maximum individual impurity less than 0.05% at a molar yield of 87.5%. ESI-MS: m/z 418.51[M+H]⁺; ¹H-NMR(125MHz, CD₃Cl): δ 8.05 (br s, 1H), 7.96 (dd, 2H), 7.61 (dd, 2H), 7.32(d, 2H), 7.25 (m, 2H), 7.15 (d, 2H), 7.12(t, 1H), 7.06 (d, 1H), 7.01 (t, 2H), 5.36 (d, 1H), 4.51 (d, 1H), 3.00(m, 1H), 1.22(d, 3H), 1.13(d, 3H).

### Example 15: Preparation of 4-(4-fluorophenyl)-2-(2-methylpropionyl)-3-phenyl -4-oxoN-phenylbutanamide

To a 250 mL three-necked flask, 10.0 g of the compound of Formula II (0.045 mol), 6.17 g of 4-fluorobenzaldehyde (0.05 mol, 1.1 eq) and 4.88 g of isobutyraldehyde (0.068 mol, 1.5 eq) were added successively, and then 50 mL of N,N-dimethylformamide was added. The mixture was stirred until it was dissolved and clear. The gas in the flask was replaced with nitrogen for 3 times. 24 mg of Cu(SbF₆)₂ (0.045 mmol, 0.001 eq) and 178 mg of the catalyst A-1 (0.23 mmol, 0.005 eq) were then added successively to the dissolved and clear solution, and the mixture was stirred until it was dissolved and clear again. The gas in the flask was replaced with nitrogen for 3 times. The flask was installed with a condenser tube, at the top of which a nitrogen bulb was equipped. The mixture was heated up to 50-60°C to react for 24 h.

The heating was stopped, and the reaction liquid was cooled to room temperature. 20 mL of a saturated ammonium chloride solution was added dropwise to quench the reaction. To the quenched reaction liquid, 100 mL of dichloromethane was added. The mixture was subjected to phase separation to remove the saturated ammonium chloride aqueous solution. The dichloromethane layer was then washed for 5 times with 100 mL of water each time. The dichloromethane layer was further washed with 50 mL of saturated brine and the brine layer was discarded.

0.5 g of silica gel was added to the dichloromethane layer, and the mixture was stirred for 30 minutes to remove residual heavy metals, and filtered. The filter residue was washed with 30 mL of dichloromethane for twice, and the dichloromethane filtrate was combined. The dichloromethane filtrate was added with 50 mL of isopropanol, and concentrated at 45-50°C under normal pressure to remove dichloromethane. The resulting suspension was cooled to 0-10°C, stirred for 1 hour, and filered. The wet cake was dired, to obtain 16.3 g of a white powder with a HPLC purity of 99.7% and a maximum individual impurity less than 0.05% at a molar yield of 86.4%.

### Example 16: Preparation of 4-(4-fluorophenyl)-2-(2-methylpropionyl)-3-phenyl -4-oxoN-phenylbutanamide

To a 250 mL three-necked flask, 10.0 g of the compound of Formula II (0.045 mol), 6.7 g of 4-fluorobenzaldehyde (0.054 mol, 1.2 eq) and 7.15 g of isobutyraldehyde (0.10 mol, 2.2 eq) were added successively, and 80 mL of tetrahydrofuran was then added. The mixture was stirred until it was dissolved and clear. The gas in the flask was replaced with nitrogen for 3 times. 48.4 mg of Cu(SbF₆)₂ (0.09 mmol, 0.002 eq) and 204 mg of the catalyst A-2 (0.27 mmol, 0.006 eq) were added successively to the dissolved and clear solution and the mixture was stirred until it was dissolved and clear again. The gas in the flask was replaced with nitrogen for 3 times. The flask was installed with a condenser tube, at the top of which a nitrogen bulb was equipped. The mixture was heated up to 50-60°C to react for 24 h.

The heating was stopped, and the reaction liquid was cooled to room temperature. 10 mL of a saturated ammonium chloride solution was added dropwise to quench the reaction. The quenched reaction liquid was concentrated at 45-55°C under reduced pressure to remove away tetrahydrofuran. To the concentrated residue, 80 mL of dichloromethane was added, and the mixture was stirred until it was dissolved and clear. The mixture was subjected to phase separation to remove the saturated ammonium chloride aqueous solution. The dichloromethane layer was then washed with 50 mL of water and the water layer was discarded. The dichloromethane layer was further washed with 50 mL of saturated brine and the brine layer was discarded.

0.5 g of silica gel was added to the dichloromethane layer, and the mixture was stirred for 30 minutes to remove residual heavy metals, and filtered. The filter residue was washed with 30 mL of dichloromethane for twice, and the dichloromethane filtrate was combined. The dichloromethane filtrate was added with 50 mL of isopropanol, and concentrated at 45-50°C under normal pressure to remove dichloromethane. The resulting suspension was cooled to 0-10°C, stirred for 1 hour, and filered. The wet cake was dired, to obtain 15.8 g of a white powder with a HPLC purity of 99.8% and a maximum individual impurity of 0.04% at a molar yield of 83.7%.

### Example 17: Preparation of 4-(4-fluorophenyl)-2-(2-methylpropionyl)-3-phenyl -4-oxoN-phenylbutanamide

To a 250 mL three-necked flask, 10.0 g of the compound of Formula II (0.045 mol), 6.7 g of 4-fluorobenzaldehyde (0.054 mol, 1.2 eq) and 6.5 g of isobutyraldehyde (0.09 mol, 2.0 eq) were added successively, and 80 mL of tetrahydrofuran was then added. The mixture was stirred until it was dissolved and clear. The gas in the flask was replaced with nitrogen for 3 times. 48.4 mg of Cu(SbF₆)₂ (0.09 mmol, 0.002 eq) and 257 mg of the catalyst A-3 (0.32 mmol, 0.007 eq) were then added successively to the dissolved and clear solution, and the mixture was stirred until it was dissolved and clear again. The gas in the flask was replaced with nitrogen for 3 times. The flask was installed with a condenser tube, at the top of which a nitrogen bulb was equipped. The mixture was heated up to 50-60°C to react for 24 h.

The heating was stopped, and the reaction liquid was cooled to room temperature. 10 mL of a saturated ammonium chloride solution was added dropwise to quench the reaction. The quenched reaction liquid was concentrated at 45-55°C under reduced pressure to remove away tetrahydrofuran. To the concentrated residue, 80 mL of dichloromethane was added, and the mixture was stirred until it was dissolved and clear. The mixture was subjected to phase separation to remove the saturated ammonium chloride aqueous solution. The dichloromethane layer was then washed with 50 mL of water and the water layer was discarded. The dichloromethane layer was further washed with 50 mL of saturated brine and the brine layer was discarded.

0.5 g of silica gel was added to the dichloromethane layer, and the mixture was stirred for 30 minutes to remove residual heavy metals, and filtered. The filter residue was washed with 30 mL of dichloromethane for twice, and the dichloromethane filtrate was combined. The dichloromethane filtrate was added with 50 mL of isopropanol, and concentrated at 45-50°C under normal pressure to remove dichloromethane. The resulting suspension was cooled to 0-10°C, stirred for 1 hour, and filered. The wet cake was dired, to obtain 16.0 g of a white powder with a HPLC purity of 99.7% and a maximum individual impurity of 0.07% at a molar yield of 84.8%.

### Example 18: Preparation of 4-(4-fluorophenyl)-2-(2-methylpropionyl)-3-phenyl -4-oxoN-phenylbutanamide

To a 250 mL three-necked flask, 10.0 g of the compound of Formula II (0.045 mol), 6.7 g of 4-fluorobenzaldehyde (0.054 mol, 1.2 eq) and 6.5 g of isobutyraldehyde (0.09 mol, 2.0 eq) were added successively, and 80 mL of tetrahydrofuran was then added. The mixture was stirred until it was dissolved and clear. The gas in the flask was replaced with nitrogen for 3 times. 48.4 mg of Cu(SbF₆)₂ (0.09 mmol, 0.002 eq) and 249 mg of the catalyst A-4 (0.32 mmol, 0.007 eq) were added successively to the dissolved and clear solution, and the mixture was stirred until it was dissolved and clear again. The gas in the flask was replaced with nitrogen for 3 times. The flask was installed with a condenser tube, at the top of which a nitrogen bulb was equipped. The mixture was heated up to 50-60°C to react for 24 h.

The heating was stopped, and the reaction liquid was cooled to room temperature. 10 mL of a saturated ammonium chloride solution was added dropwise to quench the reaction. The quenched reaction liquid was concentrated at 45-55°C under reduced pressure to remove away tetrahydrofuran. To the concentrated residue, 80 mL of dichloromethane was added, and the mixture was stirred until it was dissolved and clear. The mixture was subjected to phase separation to remove the saturated ammonium chloride aqueous solution. The dichloromethane layer was then washed with 50 mL of water and the water layer was discarded. The dichloromethane layer was further washed with 50 mL of saturated brine and the brine layer was discarded.

0.5 g of silica gel was added to the dichloromethane layer, and the mixture was stirred for 30 minutes to remove residual heavy metals, and filtered. The filter residue was washed with 30 mL of dichloromethane for twice, and the dichloromethane filtrate was combined. The dichloromethane filtrate was added with 50 mL of isopropanol, and concentrated at 45-50°C under normal pressure to remove dichloromethane. The resulting suspension was cooled to 0-10°C, stirred for 1 hour, and filered. The wet cake was dired, to obtain 15.1 g of a white powder with a HPLC purity of 99.7% and a maximum individual impurity of 0.06% at a molar yield of 80.0%.

### Example 19: Preparation of 4-(4-fluorophenyl)-2-(2-methylpropionyl)-3-phenyl -4-oxoN-phenylbutanamide

To a 250 mL three-necked flask, 10.0 g of the compound of Formula II (0.045 mol), 7.0 g of 4-fluorobenzaldehyde (0.056 mol, 1.25 eq) and 7.16 g of isobutyraldehyde (0.10 mol, 2.2 eq) were added successively, and 80 mL of tetrahydrofuran was then added. The mixture was stirred until it was dissolved and clear. The gas in the flask was replaced with nitrogen for 3 times. 48.4 mg of Cu(SbF₆)₂ (0.09 mmol, 0.002 eq) and 258 mg of the catalyst A-5 (0.32 mmol, 0.007 eq) were added successively to the dissolved and clear solution, and the mixture was stirred until it was dissolved and clear again. The gas in the flask was replaced with nitrogen for 3 times. The flask was installed with a condenser tube, at the top of which a nitrogen bulb was equipped. The mixture was heated up to 50-60°C to react for 28 h.

The heating was stopped, and the reaction liquid was cooled to room temperature. 10 mL of a saturated ammonium chloride solution was added dropwise to quench the reaction. The quenched reaction liquid was concentrated at 45-55°C under reduced pressure to remove away tetrahydrofuran. To the concentrated residue, 80 mL of dichloromethane was added, and the mixture was stirred until it was dissolved and clear. The mixture was subjected to phase separation to remove the saturated ammonium chloride aqueous solution. The dichloromethane layer was then washed with 50 mL of water and the water layer was discarded. The dichloromethane layer was further washed with 50 mL of saturated brine and the brine layer was discarded.

0.5 g of silica gel was added to the dichloromethane layer, and the mixture was stirred for 30 minutes to remove residual heavy metals, and filtered. The filter residue was washed with 30 mL of dichloromethane for twice, and the dichloromethane filtrate was combined. The dichloromethane filtrate was added with 50 mL of isopropanol, and concentrated at 45-50°C under normal pressure to remove dichloromethane. The resulting suspension was cooled to 0-10°C, stirred for 1 hour, and filered. The wet cake was dired, to obtain 15.3 g of a white powder with a HPLC purity of 99.6% and a maximum individual impurity of 0.06% at a molar yield of 81.0%.

### Example 20: Preparation of 4-(4-fluorophenyl)-2-(2-methylpropionyl)-3-phenyl -4-oxoN-phenylbutanamide

To a 250 mL three-necked flask, 10.0 g of the compound of Formula II (0.045 mol), 7.3 g of 4-fluorobenzaldehyde (0.059 mol, 1.3 eq) and 8.14 g of isobutyraldehyde (0.11 mol, 2.5 eq) were added successively, and 80 mL of tetrahydrofuran was then added. The mixture was stirred until it was dissolved and clear. The gas in the flask was replaced with nitrogen for 3 times. 72.5 mg of Cu(SbF₆)₂ (0.14 mmol, 0.003 eq) and 295 mg of the catalyst A-6 (0.36 mmol, 0.008 eq) were then added successively to the dissolved and clear solution, and the mixture was stirred until it was dissolved and clear again. The gas in the flask was replaced with nitrogen for 3 times. The flask was installed with a condenser tube, at the top of which a nitrogen bulb was equipped. The mixture was heated up to 50-60°C to react for 36 h.

The heating was stopped, and the reaction liquid was cooled to room temperature. 10 mL of a saturated ammonium chloride solution was added dropwise to quench the reaction. The quenched reaction liquid was concentrated at 45-55°C under reduced pressure to remove away tetrahydrofuran. To the concentrated residue, 80 mL of dichloromethane was added, and the mixture was stirred until it was dissolved and clear. The mixture was subjected to phase separation to remove the saturated ammonium chloride aqueous solution. The dichloromethane layer was then washed with 50 mL of water and the water layer was discarded. The dichloromethane layer was further washed with 50 mL of saturated brine and the brine layer was discarded.

0.5 g of silica gel was added to the dichloromethane layer, and the mixture was stirred for 30 minutes to remove residual heavy metals, and filtered. The filter residue was washed with 30 mL of dichloromethane for twice, and the dichloromethane filtrate was combined. The dichloromethane filtrate was added with 50 mL of isopropanol, and concentrated at 45-50°C under normal pressure to remove dichloromethane. The resulting suspension was cooled to 0-10°C, stirred for 1 hour, and filered. The wet cake was dired, to obtain 15.6 g of a white powder with a HPLC purity of 99.5% and a maximum individual impurity less than 0.06% at a molar yield of 82.7%.

## Claims

1. A method for multicomponent one-pot synthesis of the intermediate of atorvastatin calcium of formula I, comprising
reacting a compound of Formula II with 4-fluorobenzaldehyde and isobutyraldehyde under the action of Cu(SbF₆)₂ and a catalyst A by a one-pot process to synthesize a compound of Formula I: 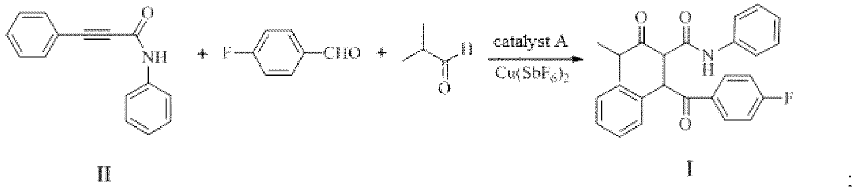
wherein the multicomponent reaction is carried out in an aprotic polar solvent which is one selected from tetrahydrofuran and N,N-dimethylformamide, or a mixed solvent thereof.

2. The method according to claim 1, wherein in the multicomponent reaction, the catalyst A is a combination of an N-hydroxyaminocyclohexylformamide derivative ligand and PdCl₂, and the N-hydroxyaminocyclohexylformamide derivative ligand has a structure represented by Formula III: 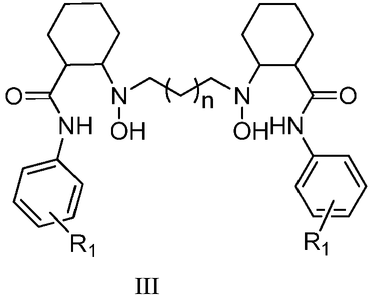
wherein n is an integer from 1-4 natural numbers;
R₁ is an electron donating group selected from methyl, methoxy, and dimethylamino.

3. The method according to claim 1, wherein in the multicomponent reaction, the N-hydroxyaminocyclohexylformamide derivative ligand in the catalyst A is synthetized by the following scheme:
(1) reacting N-tert-butoxycarbonylaminocyclohexyl-2-carboxylic acid with a substituted aromatic amine under the action of a condensing agent to prepare compound IV;
(2) removing a Boc protection from the compound IV under an acidic condition to obtain compound V;
(3) reacting the compound V with a dibromoalkane-based substance to obtain compound VI; and
(4) reacting the compound VI under the action of an oxidizing agent to obtain an N-hydroxyaminocyclohexylformamide derivative ligand; 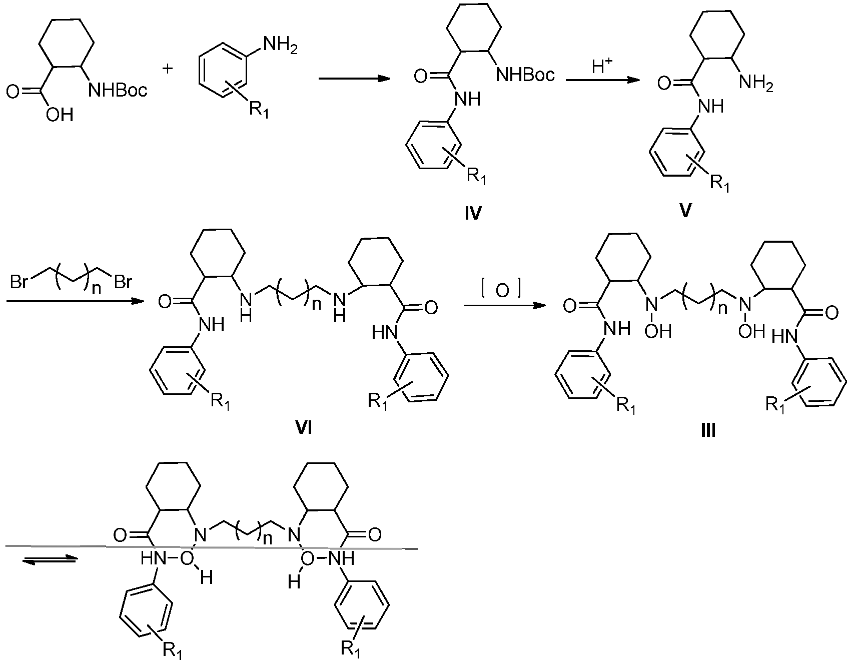
wherein n is an integer from 1-4 natural numbers;
R₁ is an electron donating group selected from methyl, methoxy, and dimethylamino.

4. The method according to claim 3, wherein in the multicomponent reaction, in the synthesis of the N-hydroxyaminocyclohexylformamide derivative ligand in the catalyst A, the condensing agent is selected from 1-propylphosphonic acid cyclic anhydride, 1-ethyl-(3-dimethylaminopropyl)carbodiimide hydrochloride, and dicyclohexylcarbodiimide; and the oxidizing agent is selected from hydrogen peroxide, tert-butyl hydroperoxide, and meta-chloroperoxybenzoic acid.

5. The method according to claim 2, wherein in the multicomponent reaction, a molar ratio of the N-hydroxyaminocyclohexylformamide derivative ligand to PdCl₂ in the catalyst A is 1.2-1.3 : 1.0.

6. The method according to claim 1, wherein in the multicomponent reaction, an amount of the catalyst A is from 0.005 to 0.008 molar equivalent relative to the compound of Formula II.

7. The method according to claim 1, wherein in the multicomponent reaction, an amount of Cu(SbF₆)₂ is from 0.001 to 0.003 molar equivalent relative to the compound of Formula II.

8. The method according to claim 1, wherein in the multicomponent reaction, an amount of 4-fluorobenzaldehyde is from 1.1 to 1.3 molar equivalent relative to the compound of Formula II; and an amount of isobutyraldehyde is from 1.5 to 2.5 molar equivalent relative to the compound of Formula II.

9. The method according to claim 1, wherein the multicomponent reaction is carried out under heating at a temperature from 50 to 60°C.

## Patentansprüche

1. Verfahren zur Mehrkomponenten-Eintopfsynthese des Zwischenprodukts von Atorvastatin-Calcium der Formel I, umfassend
Umsetzen einer Verbindung der Formel II mit 4-Fluorbenzaldehyd und Isobutyraldehyd unter der Einwirkung von Cu(SbF₆)₂ und einem Katalysator A mittels eines Eintopfprozesses, um eine Verbindung der Formel I zu synthetisieren: 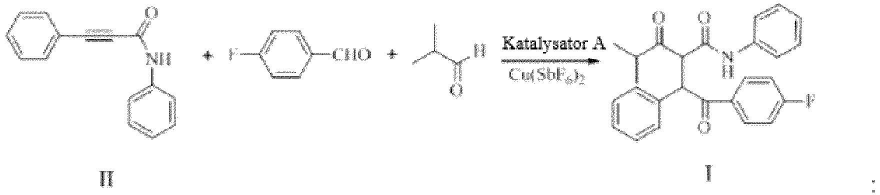
wobei die Mehrkomponentenreaktion in einem aprotischen polaren Lösungsmittel durchgeführt wird, das aus einem von Tetrahydrofuran und N,N-Dimethylformamid ausgewählt ist oder ein Lösungsmittelgemisch davon ist.

2. Verfahren nach Anspruch 1, wobei der Katalysator A in der Mehrkomponentenreaktion eine Kombination aus einem N-Hydroxyaminocyclohexylformamid-Derivatliganden und PdCl₂ ist und der N-Hydroxyaminocyclohexylformamid-Derivatligand eine durch die Formel III dargestellte Struktur aufweist: 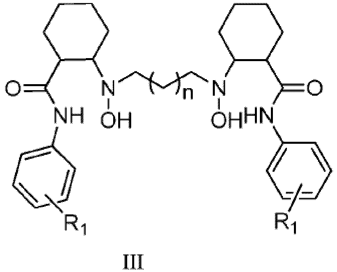
wobei n eine ganze Zahl von 1-4 ist;
R₁ eine elektronenspendende Gruppe ist, die aus Methyl, Methoxy und Dimethylamino ausgewählt ist.

3. Verfahren nach Anspruch 1, wobei der N-Hydroxyaminocyclohexylformamid-Derivatligand in dem Katalysator A in der Mehrkomponentenreaktion nach dem folgenden Schema synthetisiert wird:
(1) Umsetzen von N-tert-Butoxycarbonylaminocyclohexyl-2-carbonsäure mit einem substituierten aromatischen Amin unter der Einwirkung eines Kondensationsmittels, um Verbindung IV herzustellen;
(2) Entfernen einer Boc-Schutzgruppe aus der Verbindung IV unter sauren Bedingungen, um Verbindung V zu erhalten;
(3) Umsetzen der Verbindung V mit einer Dibromalkan-basierten Substanz, um Verbindung VI zu erhalten; und
(4) Umsetzen der Verbindung VI unter der Einwirkung eines Oxidationsmittels, um einen N-Hydroxyaminocyclohexylformamid-Derivatliganden zu erhalten; 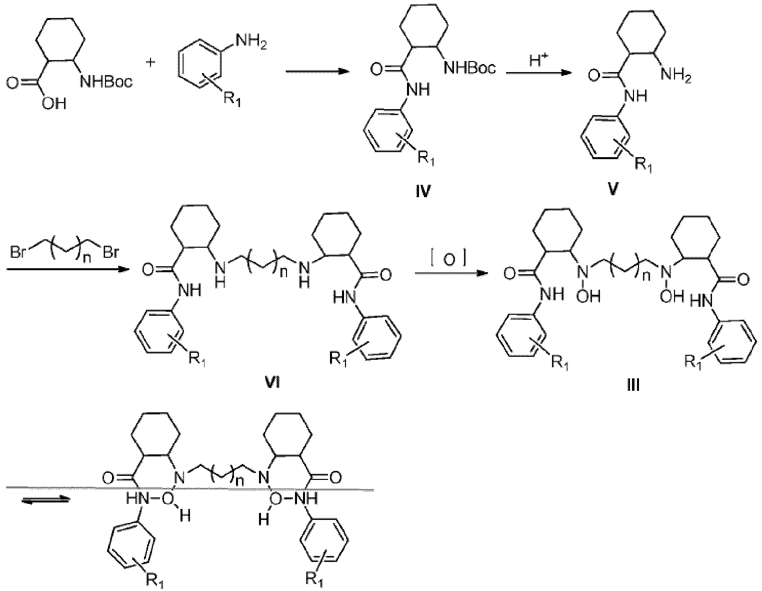
wobei n eine ganze Zahl von 1-4 ist;
R₁ eine elektronenspendende Gruppe ist, die aus Methyl, Methoxy und Dimethylamino ausgewählt ist.

4. Verfahren nach Anspruch 3, wobei das Kondensationsmittel in der Mehrkomponentenreaktion bei der Synthese des N-Hydroxyaminocyclohexylformamid-Derivatliganden in dem Katalysator A aus cyclischem 1-Propylphosphonsäureanhydrid, 1-Ethyl-(3-dimethylaminopropyl)carbodiimid-Hydrochlorid und Dicyclohexylcarbodiimid ausgewählt ist; und das Oxidationsmittel aus Wasserstoffperoxid, tert-Butylhydroperoxid und meta-Chlorperoxybenzoesäure ausgewählt ist.

5. Verfahren nach Anspruch 2, wobei in der Mehrkomponentenreaktion ein Molverhältnis des N-Hydroxyaminocyclohexylformamid-Derivatliganden zu PdCl₂ in dem Katalysator A 1,2-1,3:1,0 beträgt.

6. Verfahren nach Anspruch 1, wobei in der Mehrkomponentenreaktion eine Menge des Katalysators A 0,005 bis 0,008 Moläquivalente in Bezug auf die Verbindung der Formel II beträgt.

7. Verfahren nach Anspruch 1, wobei in der Mehrkomponentenreaktion eine Menge an Cu(SbF₆)₂ 0,001 bis 0,003 Moläquivalente in Bezug auf die Verbindung der Formel II beträgt.

8. Verfahren nach Anspruch 1, wobei in der Mehrkomponentenreaktion eine Menge an 4-Fluorbenzaldehyd 1,1 bis 1,3 Moläquivalente in Bezug auf die Verbindung der Formel II beträgt; und eine Menge an Isobutyraldehyd 1,5 bis 2,5 Moläquivalente in Bezug auf die Verbindung der Formel II beträgt.

9. Verfahren nach Anspruch 1, wobei die Mehrkomponentenreaktion unter Erwärmung bei einer Temperatur von 50 bis 60 °C durchgeführt wird.

## Revendications

1. Procédé de synthèse monotope multicomposants d'un intermédiaire calcique d'atorvastatine de formule I, comprenant
la réaction d'un composé de formule II avec du 4-fluorobenzaldéhyde et de l'isobutyraldéhyde sous l'action de Cu(SbF₆)₂ et d'un catalyseur A par un procédé monotope pour synthétiser un composé de formule I : 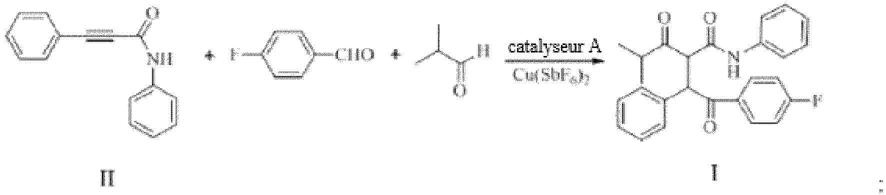
dans laquelle la réaction multicomposants est réalisée dans un solvant polaire aprotique qui est choisi parmi le tétrahydrofurane et le N,N-diméthylformamide, ou un de leurs solvants mixtes.

2. Procédé selon la revendication 1, dans lequel, dans la réaction multicomposants, le catalyseur A est une combinaison d'un ligand dérivé du N-hydroxyaminocyclohexylformamide et de PdCl₂, et le ligand dérivé du N-hydroxyaminocyclohexylformamide présente une structure représentée par la formule III : 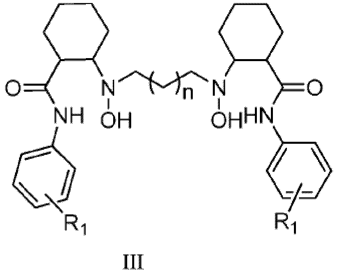
dans laquelle n est un entier de 1 à 4 entiers naturels ;
R₁ est un groupe donneur d'électrons choisi parmi méthyle, méthoxy et diméthylamino.

3. Procédé selon la revendication 1, dans lequel, dans la réaction multicomposants, le ligand dérivé du N-hydroxyaminocyclohexylformamide dans le catalyseur A est synthétisé selon le schéma suivant :
(1) faire réagir de l'acide N-tert-butoxycarbonylaminocyclohexyl-2-carboxylique avec une amine aromatique substituée sous l'action d'un agent de condensation pour préparer le composé IV ;
(2) retirer une protection Boc du composé IV dans des conditions acides pour obtenir le composé V ;
(3) faire réagir le composé V avec une substance à base de dibromoalcane pour obtenir le composé VI ; et
(4) faire réagir le composé VI sous l'action d'un agent oxydant pour obtenir un ligand dérivé du N-hydroxyaminocyclohexylformamide ; 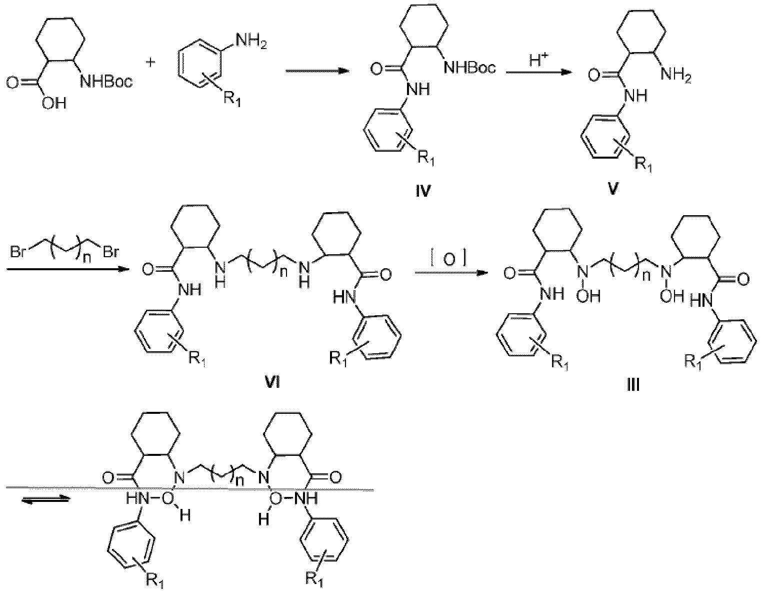
dans laquelle n est un entier de 1 à 4 entiers naturels ;
R₁ est un groupe donneur d'électrons choisi parmi méthyle, méthoxy et diméthylamino.

4. Procédé selon la revendication 3, dans lequel, dans la réaction multicomposants, dans la synthèse du ligand dérivé du N-hydroxyaminocyclohexylformamide dans le catalyseur A, l'agent de condensation est choisi parmi l'anhydride cyclique de l'acide 1-propylphosphonique, le chlorhydrate de 1-éthyl-(3-diméthylaminopropyl)carbodiimide et le dicyclohexylcarbodiimide ; et l'agent oxydant est choisi parmi le peroxyde d'hydrogène, l'hydroperoxyde de tert-butyle et l'acide méta-chloroperoxybenzoïque.

5. Procédé selon la revendication 2, dans lequel, dans la réaction multicomposants, un rapport molaire du ligand dérivé du N-hydroxyaminocyclohexylformamide à PdCl₂ dans le catalyseur A est de 1,2 à 1,3 : 1,0.

6. Procédé selon la revendication 1, dans lequel, dans la réaction multicomposants, une quantité de catalyseur A est de 0,005 à 0,008 équivalent molaire par rapport au composé de formule II.

7. Procédé selon la revendication 1, dans lequel, dans la réaction multicomposants, une quantité de Cu(SbF₆)₂ est de 0,001 à 0,003 équivalent molaire par rapport au composé de formule II.

8. Procédé selon la revendication 1, dans lequel, dans la réaction multicomposants, une quantité de 4-fluorobenzaldéhyde est de 1,1 à 1,3 équivalents molaires par rapport au composé de formule II ; et une quantité d'isobutyraldéhyde est de 1,5 à 2,5 équivalents molaires par rapport au composé de formule II.

9. Procédé selon la revendication 1, dans lequel la réaction multicomposants est réalisée par chauffage à une température de 50 à 60 °C.
